# EUROPEAN PATENT SPECIFICATION

(11) **EP 1 962 091 B1**
(45) Date of publication and mention of the grant of the patent: **08.12.2010**
(21) Application number: 08003179.2
(22) Date of filing: 21.02.2008
(51) Int. Cl.: G01N 33/53, G01N 33/50, G01N 33/536, C12Q 1/56

(54) **Methods and kits for detecting and measuring ADAMTS13/FX1 complexes**
Verfahren und Kits zur Erkennung und Messung von ADAMTS13/FX1-Komplexen
Procédés et kits pour détecter et mesurer les complexes ADAMTS13/FX1

(30) Priority: 22.02.2007 US 709562
(43) Date of publication of application: 27.08.2008
(73) Proprietor: AMERICAN DIAGNOSTICA, INC., Stamford, CT 06911-0215 (US)
(72) Inventor: Greenfield, Robert, Trumbull, CT 06611 (US); Ranzurmal, Safi, Milford, CT 06460 (US); Fryer, Hugh, New Haven, CT 06515 (US)
(74) Representative: Goddar, Heinz J.

(56) References cited:
- WO-A-2006/019431
- US-A1- 2005 186 646
- DATABASE BIOSIS [Online] BIOSCIENCES INFORMATION SERVICE, PHILADELPHIA, PA, US; November 2007 (2007-11), FRYER HUGH J L ET AL: "ADAMTS13 antigen, activity, and autoantibody and ADAMTS13/fXI complex levels in patients with venous thromboembolism" XP002475681 Database accession no. PREV200800216907
- ANDERSON PATRICIA J ET AL: "Factor XI/ADAMTS13 complexes are quantitatively insignificant in human plasma" HAEMATOLOGICA-THE HEMATOLOGY JOURNAL, vol. 92, no. 10, October 2007 (2007-10), pages 1419-1422 URL, XP002475691 ISSN: 0390-6078(print) 1592-8721(ele
- FAREED J ET AL: "POTENTIAL ROLE OF ADAMTS13/FXI COMPLEXES IN THE PATHOGENESISOF END STAGE RENAL DISEASE" BLOOD, W.B. SAUNDERS, PHILADELPHIA, VA, US, vol. 106, no. 11, PART 2, 16 November 2005 (2005-11-16), page 109B,ABSTRACT4116, XP008070653 ISSN: 0006-4971

## Description

### FIELD OF THE INVENTION

The invention provides assays for measurement of ADAMTS13 activity, detection of ADAMTS13/FXI complexes, and diagnosis of thrombotic conditions and disorders of hemostasis.

### BACKGROUND OF THE INVENTION

Hemostasis is a complex process involving platelets, coagulation factors and fibrin formation, triggered as a normal physiological response to wounding of a blood vessel. Examples of hemostatic disorders are thrombotic thrombocytopenia Purpura (TTP) and hemophilia. Thrombosis is the pathological counterpart to the normal hemostatic process, whereby a clot forms in a blood vessel and obstructs blood flow. Thrombosis can be associated with a number of underlying conditions, such as cancer, stroke, arteriosclerosis, myocardial infarction (MI) and sickle cell anemia.

Thrombotic Thrombocytopenia Purpura (TTP) is a life threatening thrombotic microangiopathic disease characterized by hemolytic anemia and thrombocytopenia associated with platelet aggregation. The cause of TTP has been recently linked to abnormalities in a metalloproteinase called ADAMTS13 or von Willebrand factor cleaving protease. ADAMTS13 is an enzyme that is present in significant levels in plasma, and may be expressed in other tissues (Levy et al. 2001, Nature 413:488-494; Plaimauer et al. 2002, Blood 100:3626-3632). Suzuki et al. (2004, Biochem. Biophys. Res. Com. 313:212-216) recently reported ADAMTS13 in platelets.

ADAMTS13 functions by cleaving ultralarge von Willebrand factor (VWF) multimers to smaller VWF proteins. Decreased VWF cleaving protease activity leads to persistence of unusually large multimers of VWF that bind to platelets, causing platelet aggregates, microangiopathic hemolysis, and thrombocytopenia in patients with TTP. Clinical manifestations of TTP are difficult to distinguish from hemolytic uremic syndrome (HUS), another thrombotic microangiopathic disorder. Recent studies indicate that low levels of ADAMTS13 activity are associated with TTP, but not HUS (Veyradier A, et al. 2002, Blood 98:1765-1772; Furlan et al. 1998, Blood 91:2839-2846). Thus, differential diagnosis of TTP can be made by measuring ADAMTS13 activity.

There are two forms of TTP - congenital (familial) and acquired (Furlan et al. 1996, Blood 87:4223-4234; Furlan et al. 1998, Blood 91:2839-2846). Congenital TTP is caused by genetic mutations in the ADAMTS13 gene, which result in a loss in ADAMTS13 production and/or the production of a non-functional ADAMTS13 enzyme. Acquired TTP is an autoimmune-like disease, which has been linked to intake of certain pharmaceutical drugs. Acquired TTP is caused by the generation of autoantibodies to ADAMTS13 protein. The onset of acquired TTP has been linked to intake of certain pharmaceutical drugs.

Several methods for measuring the presence and/or activity of ADAMTS13 are known in the art. These methods include collagen binding assays, ristocetin cofactor assays, electrophoretic analysis (*e.g*. multimer analysis) and immunological methods. Electrophoretic immunoassays, such as Western blotting analysis, have largely been replaced by immunoradiometric assays (IRMA) and enzyme-linked immunosorbant assays (ELISA) (Laffan et al. 2004, Haemophilia 10:199-217). Several reports describe ADAMTS13 assays where the A2 domain of VWF is used as a substrate (Zhou et al. 2004, Thromb. Haemost. 91:806-811; Kokame et al. 2004, Hemost. Thromb. Vasc. Biol. Blood 103:607-612; Cruz et al. 2004, Thromb. Haemost. 90:1204-1209). Cleavage of the A2 domain is monitored by an ELISA method. The available assays for ADAMTS13 require a relatively high technical skill level, and are therefore not performed in most clinical laboratories. In addition, obtaining results can take several days using available tests, which is detrimental to patients presenting with TTP or other hemostatic or thrombotic conditions, who require rapid diagnosis.

Simple, specific and rapid assays for measuring ADAMTS13 and ADAMTS13-related moieties are needed enhance diagnosis of thrombotic and hemostatic conditions and aid patient care. Attempts at developing ADAMTS13 activity assays have been difficult. Furlan *et al.* tested 28 synthetic chromogenic peptidyl substrates with para-nitroanaline (pNA) as the leaving group, and did not observe consistent, repeatable results (2002 Seminars in Thrombosis and Hemostasis 28(2): 167-172). These results demonstrate the difficulty in the art of developing a rapid, reliable assay for ADAMTS13 activity. More recently several ADAMTS13 activity assays have been described including collagen binding assay, vWF cleavage assay, ELISA assays using VWF73 peptidyl substrate and fluorescence resonance energy transfer (FRET) assays.

WO2006/019431 describes a method for diagnosing thrombotic thrombocytopenia purpura (TTP) by detecting ADAMTS13/FXI complexes in a sample, comprising binding an anti-ADAMTS13 antibody to a surface, contacting the sample with said surface, adding an anti-FXI antibody, and detecting the ADAMTS13/FXI complex.

We have developed diagnostic chromogenic assays for measuring ADAMTS13 activity on platelets and in plasma using different peptidyl substrates with leaving groups other than pNA. We have also developed an assay method to measure autoantibodies to ADAMTS13 and an ELISA for measuring ADAMTS13 protein in plasma.

Moreover, we have discovered that platelet ADAMTS13 binds FXIa. We provide an ADAMTS13 activity assay based upon the binding of FXIa to ADAMTS13 on platelets by measuring platelet-bound FXIa activity.

We also provide, for the first time, the identification and quantitation by ELISA of soluble ADAMTS13/FXI complexes in plasma. The levels of ADAMTS13/FXI complexes can be indicative of the presence of thrombi and is diagnostic for various thrombotic conditions. We have discovered that the form of ADAMTS13 in the ADAMTS13/FXI complex in plasma is a proteolytic fragment of full-length ADAMTS13 and that proteolysis or disruption of the native ADAMTS13 structure by binding to a solid surface is required for formation of the ADAMTS13/FXI complex *in vitro*.

### SUMMARY OF THE INVENTION

The present invention provides a method for detecting a pulmonary embolism as defined in the appended claims.

In one aspect, there is also disclosed a method for measuring ADAMTS13 activity, not forming part of the present invention, comprising (a) incubating a sample comprising ADAMTS13 with a substrate, wherein the substrate comprises a peptide moiety and a chromogenic or fluorogenic moiety, wherein the peptide moiety comprises X-Val-Tyr, X-Leu-Tyr or X-Ile-Tyr, wherein X is any amino acid, and wherein the chromogenic moiety is not para-nitroanaline (pNA); and (b) measuring optical density or fluorescence of the sample; thereby measuring ADAMTS13 activity.

In a second aspect there is also disclosed a method for measuring ADAMTS13 activity, not forming part of the present invention, comprising (a) incubating a sample comprising ADAMTS13 with a substrate, wherein the substrate comprises X-peptide moiety-Z, wherein the peptide moiety comprises Val-Tyr-Met, Leu-Tyr-Met or Ile-Tyr-Met, wherein X is a donor moiety and Z is an acceptor moiety, and wherein the donor and acceptor moieties mediate-fluorescence resonance energy transfer; and (b) measuring fluorescence of the sample; thereby measuring ADAMTS13 activity.

There is also disclosed an ADAMTS13 protein isolated from platelets, not forming part of the present invention, wherein the ADAMTS13 protein is cleaved into more than one peptide, and wherein at least one cleaved peptide has a molecular weight on an SDS-PAGE gel of about 120 kD or less than 120 kD.

There is also disclosed a method for making an ADAMTS13 substrate for measuring ADAMTS13 activity comprising covalently linking a peptide moiety to a chromogenic or fluorogenic moiety, wherein the peptide moiety comprises X-Val-Tyr, X-Leu-Tyr or X-Ile-Tyr, wherein X is any amino acid, and wherein the chromogenic moiety is not pNA.

In a fifth aspect, there is also disclosed a method for making an ADAMTS13 substrate for measuring ADAMTS13 activity, not forming part of the present invention, comprising covalently linking a donor moiety, a peptide moiety and an acceptor moiety sequentially, wherein the peptide moiety comprises Val-Tyr-Met, Leu-Tyr-Met or Ile-Tyr-Met, and wherein the donor and acceptor moieties mediate fluorescence resonance energy transfer.

There is also disclosed a kit, not forming part of the present invention, comprising a substrate for measuring ADAMTS13 activity, wherein the substrate comprises a peptide moiety as described herein and a chromogenic or fluorogenic moiety. Alternatively, the substrate comprises donor moiety, a peptide moiety as described herein, and an acceptor moiety, wherein the donor and acceptor moieties mediate fluorescence resonance energy transfer.

In another aspect, there is also disclosed a method for inhibiting ADAMTS13 activity, not forming part of the present invention, comprising incubating a sample comprising ADAMTS13 with an inhibitory antibody against ADAMTS13.

In a further aspect, there is also disclosed a method for identifying an inhibitor of ADAMTS13, not forming part of the present invention, comprising (a) incubating a sample comprising ADAMTS13 with a candidate inhibitor of ADAMTS13; and measuring ADAMTS13 activity in the test sample according to the method of the first or second aspect as disclosed above; wherein the inhibitor is identified by reduced ADAMTS13 activity in the sample compared with ADAMTS13 activity in a control sample comprising ADAMTS13, wherein the control sample was not incubated with the candidate inhibitor.

There is also disclosed a method for measuring the amount of ADAMTS13 in a sample, not forming part of the present invention, comprising (a) binding anti-ADAMTS13 antibody to a solid phase; (b) adding the sample to the solid phase, wherein ADAMTS13 present in the sample binds to the antibody; (c) detecting bound ADAMTS13 using direct or indirect immunolabelling; and (d) quantifying ADAMTS13 detected; thereby measuring the amount of ADAMTS13 in the sample.

There is also disclosed a method for detecting anti-ADAMTS13 antibodies in a test sample, not forming part of the present invention, comprising (a) binding an anti-ADAMTS13 antibody raised in a first species to a solid phase; (b) adding ADAMTS13 to the solid phase, wherein the ADAMTS13 binds to the antibody raised in the first species; (c) adding the test sample to the solid phase, wherein the sample is from a second species and wherein anti-ADAMTS13 antibodies present in the test sample bind to the ADAMTS13; (d) adding a labeled antibody against antibodies from the second species, wherein the labeled antibody is raised in a third species, wherein the labeled antibody binds to the anti-ADAMTS13 antibodies bound to the ADAMTS13; and (e) detecting the labeled antibody; thereby detecting anti-ADAMTS13 antibodies in the test sample.

In another aspect, there is also disclosed a method for diagnosing TTP in a subject, not forming part of the present invention, comprising (a) measuring ADAMTS13 activity in a test sample from the subject according to the method of the first or second aspect of the invention; and (b) comparing the ADAMTS13 activity in the test sample to ADAMTS13 activity in a control sample having normal ADAMTS13 activity; wherein TTP is diagnosed by reduced ADAMTS13 activity in the test sample compared with the control sample.

In yet another aspect, there is also disclosed a method for diagnosing acquired TTP in a subject, not forming part of the present invention, comprising (a) incubating a sample comprising ADAMTS13 with plasma from the subject; and (b) measuring ADAMTS13 activity in the sample according to the method of the first or second aspect of the invention; wherein acquired TTP is diagnosed by reduced ADAMTS13 activity in the sample compared with ADAMTS13 activity in a control sample having normal ADAMTS13 activity.

There is also disclosed a method for monitoring treatment of a patient with TTP, not forming part of the present invention, comprising measuring ADAMTS13 activity according to the first or second aspect of the invention during treatment of the patient.

There is also disclosed a method for detecting ADAMTS13/FXI complexes in a sample, not forming part of the present invention, comprising (a) binding an anti-ADAMTS13 antibody to a solid phase; (b) adding the sample to the solid phase, wherein the ADAMTS13/FXI complexes present in the sample bind to the antibody; (c) adding an anti-FXI antibody to the solid phase; and (d) detecting the ADAMTS13/FXI complexes by direct or indirect immunolabelling of the anti-FXI antibody.

In another aspect, there is also disclosed a method for measuring the.amount of ADAMTS13/FXI complexes in a sample, not forming part of the present invention, comprising (a) binding an anti-ADAMTS13 antibody to a solid phase; (b) adding the sample to the solid phase, wherein the ADAMTS13/FXI complexes present in the sample bind to the antibody; (c) adding an anti-FXI antibody to the solid phase; (d) detecting the ADAMTS13/FXI complexes by direct or indirect immunolabelling of the anti-FXI antibody; and (e) quantifying the anti-FXI antibody detected, thereby measuring the amount of ADAMTS13/FXI complexes in the sample.

The invention is a method for detecting a pulmonary embolism in a subject comprising (a) adding a test plasma sample from an individual to a solid phase having an anti-ADAMTS13 antibody bound to it, wherein the ADAMTS13/FXI complexes present in the test sample bind to the antibody; (b) adding an anti-FXI antibody to the solid phase, wherein the FXI antibody binds to FXI in the complexes; (c) and detecting the ADAMTS13/FXI complexes by direct or indirect immunolabelling of the anti-FXI antibody. The anti-FXI antibody can be quantified by procedures known in the art, such as densitometry. An increase or decrease in the amount of ADAMTS13/FXI complexes in the test sample compared with the control sample indicates a thrombotic or abnormal hemostatic condition.

There is also disclosed a kit for detecting ADAMTS13/FXI complexes in a sample, not forming part of the present invention. The kit comprises (i) a solid phase coated with an anti-ADAMTS13 antibody and (ii) an anti-FXI antibody. In a preferred embodiment, the anti-FXI antibody is labeled. In a particularly preferred embodiment, the anti-FXI antibody is conjugated to horseradish peroxidase (HRP). Optionally, the kit can include an HRP substrate. The kit can also contain a sample for devising a standard curve of ADAMTS13/FXI complex concentration. The standard sample can be any of the samples described herein, including biological fluid, whole blood, platelet rich plasma (PRP), platelet poor plasma (PPP), pooled normal plasma (PNP), washed platelets, tissue culture supernatant and recombinant proteins. The standard sample can be serially diluted by the end user to obtain a standard curve. Other optional components of the kit include a wash buffer, and a positive control. The positive control can also be any of the samples described herein.

In an especially preferred embodiment, the kit comprises (i) a plate coated with an anti-ADAMTS13 antibody; (ii) an anti-FXI antibody labeled witch HRP; (iii) an HRP substrate; (iv) a standard sample; (v) a positive control; and (vi) a wash buffer.

In a further aspect, there is also disclosed a method of regulating the activity of ADAMTS13 and/or FXI and/or FXIa, not forming part of the present invention, by enhancing or inhibiting formation of ADAMTS13/FXI complexes.

There are also disclosed anti-ADAMTS13 antibodies, in particular, monoclonal anti-ADAMTS13 antibodies MAbs 4, 5, 6, 7 and 12. Hybridomas producing the monoclonal antibodies were deposited at the American Type Culture Collection under Accession No. on . The anti-ADAMTS13 antibodies recognize the ∼80 kD ADAMTS13 fragment comprised in the ADAMTS13/FXI complex in plasma.

### BRIEF DESCRIPTION OF THE DRAWINGS

The present invention will now be described by way of example, in which reference will be made to the following Figures in which:
**Figure 1** shows the structure of the plasma form of ADAMTS13. S: signal peptide; P: propeptide; Dis: disintegrin-like region; Cys: Cys-rich region. The catalytic (metalloprotease) region and the two CUB domains are also shown. Thrombospondin type I (TSP1) repeats are shown as numbered circles.
**Figure 2** shows LVY-pNA as a substrate for ADAMTS13.
**Figures 3** shows a comparison of LVY-AMC and Suc-LLVY-AMC (SEQ ID NO: 2) substrates for measuring ADAMTS13 activity.
**Figure 4** shows the measurement of cleavage of an ADAMTS13 substrate using fluorescence resonance energy transfer (FRET). The substrate, NH₂-Arg-Lys(*DABCYL)-*NLVYMVTGD(*EDANS)*-Arg-COOH (SEQ ID NO: 1), was incubated with normal platelets, and the increase in fluorescence over time was measured.
**Figure 5** shows ADAMTS13 activity in tissue culture fluid from mock transfected and ADAMTS13 transfected HEK 293 cells. Activity was measured using Suc-LLVY-AMC (SEQ ID NO: 2) fluorescent substrate.
**Figure 6** shows a comparison of ADAMTS13 activity in plasma from normal subjects and from subjects with acquired TTP using Suc-LLVY-AMC (SEQ ID NO: 2) fluorescent substrate.
**Figure 7** shows the quantitation of ADAMTS13 activity in PRP from normal subjects (■) and subjects with acquired TTP (◆).
**Figure 8** shows the measurement of ADAMTS13 activity in platelet rich plasma (PRP) and platelet poor plasma (PPP) from six individual samples taken from normal subjects.
ADAMTS13 activity was measured using Suc-LLVY-AMC (SEQ ID NO: 2) fluorescent substrate.
**Figure 9** shows a comparison of ADAMTS13 activity per mg of protein on platelets from normal subjects and subjects with acquired TTP.

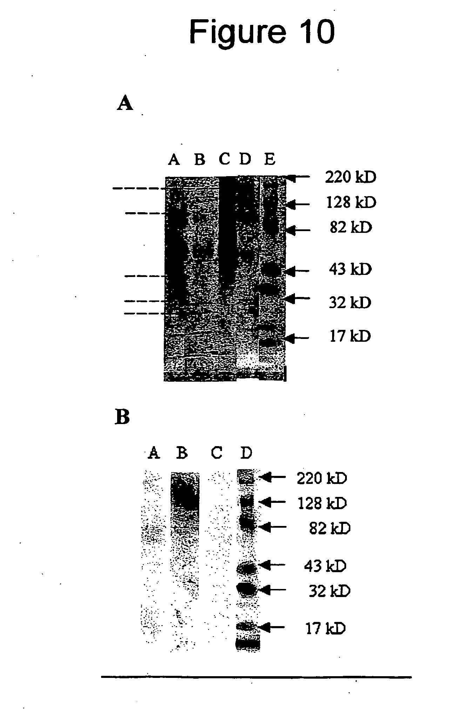

**Figure 10** shows immunostaining Western blots of platelet proteins by various anti-ADAMTS13 antibodies. Figure 10A shows platelet proteins electrophoresed using reducing conditions (lanes A and B) and non-reducing conditions (lanes C and D). Lanes A and C were stained with goat anti-PAI-1 antibody as a control; Lanes B and D were stained with goat anti-ADAMTS13 antibody; Lane E contains molecular weight standards. Arrows on the left indicate protein bands specifically immunostained by anti-ADAMTS13 antibodies. **Figure 10B** shows immunostaining of platelet proteins using human antibodies. Lane A: reducing conditions and Ig from a subject with acquired TTP; Lane B: non-reducing conditions and Ig from a subject with acquired TTP; Lane C: non-reducing conditions and Ig from a normal subject (control); Lane D: molecular weight markers.
**Figure 11** shows that ADAMTS13 activity on normal platelets and on platelets from a subject with acquired TTP is enhanced by treatment with the peptidase activated Factor XIa (FXIa).
**Figure 12** shows a plot of FXIa activated platelet ADAMTS13 activity in normal plasma, and its inhibition by plasma from a subject with acquired TTP.
**Figure 13** shows a standard curve depicting concentration dependent increase in optical density using the ADAMTS13 ELISA and different amounts of normal plasma.
**Figure 14** shows the determination of ADAMTS13 levels, relative to pooled normal plasma (PNP), in twenty-four individual plasma samples from normal subjects using the ELISA method.
**Figure 15** shows inhibition of recombinant ADAMTS13 activity by goat anti-ADAMTS13 antibody.
**Figure 16** shows recombinant ADAMTS13 activity in the presence of varying amounts of normal plasma or plasma from subjects with acquired TTP, which contains an inhibitor of the enzyme.
**Figure 17** shows inhibition of ADAMTS13 activity on isolated platelets by anti-ADAMTS13 antibodies from different species.
**Figure 18** shows the results of an experiment measuring anti-ADAMTS13 autoantibodies in plasma by preincubating sample plasma with isolated normal platelets.
**Figure 19** shows concentration dependent inhibition of platelet ADAMTS13 activity by human ADAMTS13 antibodies in plasma from subjects with acquired TTP.
**Figure 20** shows the effect of using different amounts of platelets in the fluorescence assay of the invention. ADAMTS13 activity was measured in PRP from normal subjects and subjects with acquired TTP.
**Figure 21** shows a standard curve using an ELISA of the invention to measure the level ADAMTS13/FXI complexes in various concentrations of normal plasma.
**Figure 22** shows complex formation between recombinant ADAMTS13 and FXI immobilized on a microtiter plate.
**Figure 23** shows elevated levels of ADAMTS13/FXI complex in plasma from TIP patients compared to normal donors using the ELISA with the goat anti-ADAMTS13 antibody for capture.
**Figure 24** shows the level of ADAMTS13/FXI complexes in FXI-deficient plasma, compared with normal plasma.
**Figure 25** shows that increasing amounts of ADAMTS13/FXI complexes were formed in FXI-deficient plasma upon addition of increasing amounts of FXI.
**Figure 26** shows quantitation of ADAMTS13/FXI complex in plasma from normal (n=48) donors and patients (n=18) with pulmonary embolism (PE).

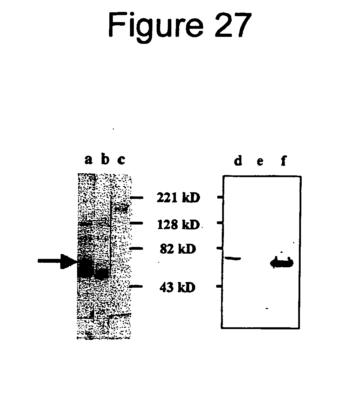

**Figure 27** shows results of immunoprecipitation of ADAMTS13/FXI complexes from plasma. Lanes a-c were detected using rabbit anti-ADAMTS13 antibody and goat anti-rabbit IgG-HRP. Lane a shows immunodetection of ADAMTS13. Lane b shows a control immunostain using no primary antibody. Lane c shows a positive control using recombinant ADAMTS13. Lanes d-f were stained using rabbit anti-FXI IgG-HRP. Lane d shows immunodetection of FXI. Lane e shows a control immunostain using no primary antibody. Lane f shows a positive control using recombinant FXI. The arrow indicates an 80 kD protein specifically detected with the rabbit anti-ADAMTS13 antibody.
**Figure 28** shows measurement of ADAMTS13/FXI complex using an ELISA with monoclonal anti-ADAMTS13 as capture. Plasma containing ADAMTS13/FXI complex was serially diluted 1:2 starting at 25% and applied to Mab 5 coated microwells. ELISA procedure was carried out according to methods in Example 16.
**Figure 29** shows frequency distribution of ADAMTS13/FXI complex in normal samples measured by ELISA using Mab 5 as capture.

### DETAILED DESCRIPTION

In this disclosure, "comprises," "comprising," "containing," "having" and the like can have the meaning ascribed to them in U.S. Patent law and can mean "includes," "including," and the like. "Consisting essentially of" or "consists essentially of" likewise have the meaning ascribed in U.S. Patent law and the term is open-ended, allowing for the presence of more than that which is recited so long as basic or novel characteristics of that which is recited is not changed by the presence of more than that which is recited, but excludes prior art embodiments.

### ADAMTS13

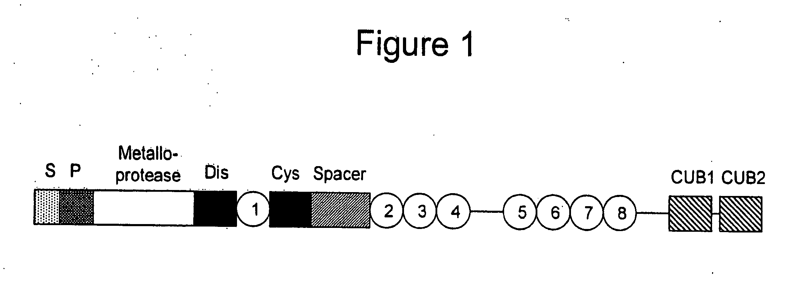

ADAMTS13, also known as von Willebrand factor (VWF)-cleaving protease, is a member of the family of metalloproteases named for the characteristic combination of a disintegrin-like and metalloprotease (reprolysin-type), with thrombospondin type 1 motifs. The structure of plasma ADAMTS13 is shown in Figure 1. Structural details and sequence information on ADAMTS13 can be found in Zheng et al. (2001; J. Biol. Chem. 276(44):41059-41063), incorporated herein by reference. See, in particular, Figure 1 of Zheng *et al.* ADAMTS13 cleaves VWF at the Tyr¹⁶⁰⁵-Met¹⁶⁰⁶ bond and requires both calcium and zinc ions to function. The sequence of human ADAMTS13 is provided as SEQ ID NO: 4.

Until the instant invention, ADAMTS13 activity was primarily studied in plasma. This form of ADAMTS13 is referred to herein as "plasma ADAMTS13". A novel form of ADAMTS13 has now been discovered on platelets and is referred to herein as "platelet ADAMTS13". "Recombinant ADAMTS13" can also be made using standard molecular biology techniques, such as those found in Sambrook, et al., Molecular Cloning, A Laboratory Manual (1989) and Ausubel et al., Short Protocols in Molecular Biology (1999) 4th Ed., John Wiley & Sons, Inc. (as well as the complete version of Current Protocols in Molecular Biology).

Therefore, there is disclosed an ADAMTS13 protein and ADAMTS13 activity in platelets. This protein appears to be distinct from plasma ADAMTS13 in that plasma ADAMTS13 is one contiguous polypeptide, whereas platelet ADAMTS13 protein is cleaved into more than one polypeptide, which is then held together by disulfide bonds. Polypeptides of about 120 kD, about 84 kD, about 60 kD, about 43 kD and about 30 kD are seen on an SDS-PAGE gel of platelet ADAMTS13. Platelet ADAMTS13 appears to be cleaved by FXIa. Likewise, this form of ADAMTS13 also appears to be distinct from that reported by Suzuki et al. (2004, Biochem. Biophys. Res. Common. 313:212-216). Unlike the cleaved platelet ADAMTS13, the ADAMTS13 of Suzuki *et al*. has a molecular weight of about 220 kD on an SDS-PAGE gel. Moreover, Suzuki *et al.* report the larger form of ADAMTS13 in platelets, while experiments performed by us indicate ADAMTS13 activity on the surface of platelets. Without wishing to be bound by theory, it is possible that the platelet ADAMTS13 has undergone different post-translational modification than that of Suzuki *et al.,* resulting in a cleaved surface protein, rather than a larger cytoplasmic protein.

The assays disclosed can be used to detect platelet ADAMTS13, plasma ADAMTS13 and recombinant ADAMTS13.

Sources of ADAMTS13 for use in the assays disclosed and in the method of the invention can be platelet rich plasma (PkP), platelet poor plasma (PPP), pooled normal plasma (PNP), isolated platelets, whole blood, tissue culture supernatant or purified ADAMTS13. Methods for making PRP, PPP, isolated platelets and tissue culture supernatant can be found in the Examples section. Purified ADAMTS13 can be made from plasma, platelets or recombinant cells using standard biochemical techniques for protein isolation and purification including, but not limited to, immunoaffinity chromatography, size exclusion chromatography, ion exchange chromatography, immunoprecipitation, and ammonium sulfate precipitation. The term "plasma" can include PRP, PPP and PNP.

As used herein, "normal platelets", "normal plasma", "normal PRP", *etc*. are derived from individuals who do not have either congenital TTP or acquired TTP. PNP is a mixture of plasma taken from multiple individuals who do not have TTP. Likewise, "TTP platelets", "TTP plasma", "TTP PRP", *etc.* are derived from individuals who have either congenital TTP or acquired TTP. "Acquired TTP platelets", "acquired TTP plasma", "acquired TTP PRP", *etc.* are derived from individuals who have the acquired form of TTP.

Measurements of ADAMTS13 activity are made in relation to "normal activity", that is, ADAMTS13 activity in normal platelets, normal plasma, normal PRP, recombinant or purified ADAMTS13 *etc.* Thus, terms such as "reduced ADAMTS13 activity" or "inhibited ADAMTS13 activity" refer to ADAMTS13 activity relative to activity measured in a normal sample, *i.e.* normal platelets, normal plasma, normal PRP, recombinant or purified ADAMTS13 *etc*., wherein the normal platelets or plasma are from an individual who does not have a hemostatic disorder or a thrombotic condition.

The method of the invention is applicable to clinical, veterinary and/or research applications.

### Factor XI

Factor XI (FXI) is a zymogen of the plasma serine protease family that, upon activation, participates in both fibrin formation and fibrinolysis (von dem Borne et al. 1995, Blood 86:3035-3042). FXI is comprised of 2 identical 80-kD polypeptides connected by a single disulfide bond. The protein has an N-terminal non-catalytic heavy chain and a C-terminal trypsin-like catalytic light chain. The heavy chain consists of 4 homologous subunits called "apple" domains, a feature that FXI shares with the closely related prekallikrein (Gailani et al. 2001, Blood 17:3117-3122).

FXI is activated *in vitro* by activated Factor XII (FXIIa), thrombin, and activated Factor XI (FXIa) (Gailani. et al. 1991, Science 253:909). Evidence strongly suggests that FXI and FXIa bind to activated platelets in a manner that requires the protein cofactor high molecular weight kininogen, and zinc ions (Sinha et al. 1984, J. Clin. Invest. 73:1550-1559). FXI and FXIa both bind to activated platelets (Greengard, J. et al (1986) Biochemistry 25: 3884-3890).

We have found that both FXI and FXIa bind to ADAMTS13 or a fragment of ADAMTS13. Adsorption of ADAMTS13 onto to a solid surface promotes binding of FXI and FXIa to the ADAMTS13 molecule. A complex is thereby formed between the adsorbed ADAMTS13 and FXI or FXIa. The invention comprehends both ADAMTS13-FXI complexes and ADAMTS13/FXIa complexes.

Without wishing to be bound by theory, we believe that cleavage of ADAMTS13 by protease(s) (*e.g*. thrombin, plasmin, FXIa and/or FXI) enhances the ability of the ADAMTS13 protein to bind to FXI or FXIa. FXI and FXIa preferentially bind to a proteolytic fragment of ADAMTS13.

The invention provides methods of detecting ADAMTS13/FXI complexes in plasma and in other biological samples. Detection and quantitation of complexes can be diagnostic for TTP, various thrombotic conditions and other hemostatic disorders.

Sources of FXI for use in the assays and methods of the invention can be platelet rich plasma (PRP), platelet poor plasma (PPP), pooled normal plasma (PNP), isolated platelets, whole blood, tissue culture supernatant or purifies FXI. Purified FXI can be made from plasma, platelets or recombinant cells using standard biochemical techniques for protein isolation and purification including, but not limited to, immunoaffinity chromatography, size exclusion chromatography, ion exchange chromatography, immunoprecipitation, and ammonium sulfate precipitation. As used herein, "FXI-deficient plasma" is derived from individuals with a congenital or acquired FXI deficiency.

### Substrates

There are disclosed assays for measuring ADAMTS13 activity. In one such assay, a sample comprising ADAMTS13 is incubated with a substrate that comprises a peptide moiety and a chromogenic or fluorogenic moiety and the optical density or fluorescence of the sample is measured, thereby measuring ADAMTS13 activity. In this embodiment, the peptide moiety comprises X-Val-Tyr, X-Leu-Tyr or X-Ile-Tyr, wherein X is any amino acid. In a preferred embodiment, X is Leu. In other preferred embodiments, the peptide moiety is Leu-Val-Tyr, Leu-Leu-Val-Tyr (SEQ ID NO: 2) or Suc-Leu-Leu-Val-Tyr (SEQ ID NO: 2).

The skilled artisan can make amino acid substitutions in the peptide moiety without undue experimentation. For example, amino acid substitutions may be made on the basis of similarity in polarity, charge, solubility, hydrophobicity, hydrophilicity, and/or the amphipathic nature of the reside as long as the binding activity of the substrate is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine.

Conservative substitutions may be made, for example, according to the Table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

Preferably, the peptide moiety is at least about 3 amino acid residues in length. More preferably, the peptide moiety is in the range of about 3 to about 20 amino acid residues in length. Non-conventional amino acids such as those found in the Spectrozyme series of peptidyl substrates from American Diagnostica Inc. (Stamford, CT) may also be used to substitute for conventional amino acids, provided that the peptide retains its ability to bind to and be cleaved by ADAMTS13.

Chromogenic moieties suitable for use in the invention include s-benzyl, 5-amino-2-nitrobenzoic acid and 6-amino-1-naphthalenesulfonamides. The chromogenic moiety is not para-nitroanaline (pNA), which has been demonstrated to be an ineffective moiety for an ADAMTS13 substrate. Without wishing to be bound by theory, it is possible that the structure of pNA creates a steric hindrance that prevents ADAMTS13 activity.

Fluorogenic moieties suitable for use in the invention include coumarins, fluoresceins, rhodamines, resorufins and dimethylacridinones. In a preferred embodiment, the fluorogenic moiety is a coumarin. In a particularly preferred embodiment, the coumarin is 7-amino-4-methylcoumarin (AMC).

Another assay is a method for measuring ADAMTS13 activity by incubating a sample comprising ADAMTS13 with a substrate having donor and acceptor moieties that mediate fluorescence resonance energy transfer (FRET). In addition to the donor and acceptor moieties, the substrate comprises a peptide moiety that comprises Val-Tyr-Met, Leu-Tyt-Met or Ile-Tyr-Met. ADAMTS13 activity is determined by measuring the fluorescence of the sample.

FRET is a distance-dependent interaction between the electronic excited states of two dye molecules in which excitation is transferred from a donor moiety to an acceptor moiety without emission of a photon. This is accomplished by using donor/acceptor pairs in which the emission spectrum of the donor overlaps the absorption spectrum of the acceptor. When the two are in spatial proximity, the excitation energy of the donor is transferred to the acceptor through long-range dipole-dipole interactions. When energy transfer occurs, the acceptor quenches the fluorescence of the donor, and thus, the acceptor moiety is also called a quencher. The donor and acceptor moieties must be within about 100 angstroms or 10 nm of one another for efficient energy transfer.

Suitable donor/acceptor pairs for use in FRET are well known in the art. Examples of donor/acceptor pairs can be found in Table 1. It should be noted that Table 1 does not contain an exhaustive list of FRET donor/acceptor pairs, and that the skilled artisan can choose a donor/acceptor pair based on his or her knowledge of the art.

**Table 1. FRET Donor and Acceptor Moieties**

| **Donor** | **Acceptor** |
|---|---|
| 7-Methoxycoumarin | DABCYL |
| Amino Methyl Coumarin | DABCYL, QSY-35¹ |
| Blue Fluorescent Protein | Ds Red Fluorescent Protein |
| BODIPY-FL² | DABCYL, QSY-7, QSY-9, BHQ-1³ |
| B-Phycoerythrin | Cy5 |
| Carboxyfluorescein Succinimidyl Ester | Texas Red |
| Cascade Blue | DABCYL |
| Coumarin | DABCYL |
| Cy3 | Cy5, QSY-7, QSY-9, BHQ-2 |
| Cy5 | Cy5.5, QSY-35, BHQ-2 |
| Dansyl | FITC |
| Dansyl | Octadecylrhodamine |
| Dialkylaminocoumarin | DABCYL, QSY-35 |
| EDANS⁴ | DABCYL |
| FITC | Eosin Thiosemicarbazide |
| Fluorescein | Tetramethylrhodamine |
| Green Fluorescent Protein | Yellow Fluorescent Protein |
| IAEDANS⁵ | DDPM⁶ |
| Marina Blue | DABCYL, QSY-35 |
| Pacific Blue | DABCYL, QSY-35 |
| Rhodamine 6G | Malachite Green |
| Tryptophan | Dansyl |

| | |
|---|---|
| ¹ QSY quenchers are analogs of fluorescein. ² 4,4-difluoro-4-bora-3a,4a-diaza-s-indacene ³ Black Hole Quenchers™ by Biosearch Technologies, Inc., Novato, CA ⁴ 5-[(2-aminoethyl)amino]naphthalene-1-sulfonic acid ⁵ 5-(2-iodoacetylaminoethyl)aminonaphthalene-1-sulfonic acid ⁶ N-(4-dimethylamino-3,5-dinitrophenyl)maleimide | |

The preferred donor/acceptor pair is EDANS/DABCYL.

Preferably, the peptide moiety is at least about 3 amino acid residues in length. More preferably, the peptide moiety is in the range of about 3 to about 30 amino acid residues in length. Advantageously, the peptide moiety is Asn-Leu-Val-Tyr-Met-Val-Thr-Gly-Asp (SEQ ID NO: 3). Amino acid substitutions can be made as described above.

The preferred substrate for use is NH₂-Arg-Lys-(*DABCYL*)- Asn-Leu-Val-Tyr-Met-Val-Thr-Gly-Asp-(*EDANS*)-Arg-COOH (SEQ ID NO: 1).

Methods for making the ADAMTS13 substrates are also contemplated. Said methods comprise covalently linking a peptide moiety, as described above, to a chromogenic or fluorogenic moiety, or to a donor moiety and an acceptor moiety that mediate FRET, using well known synthesis techniques.

### Inhibitors of ADAMTS13

There is also disclosed a method for inhibiting ADAMTS13 using an inhibitory antibody against ADAMTS13. Several anti-ADAMTS13 antibodies are available from various sources. For instance, Examples 12 and 14 demonstrate the inhibitory effects of anti-ADAMTS13 antibodies from goat, rabbit and human.

Autoantibodies that inhibit ADAMTS13 activity can be detected in subjects with acquired TTP. (See Klaus et al. 2004, Blood 103(12):4514-4519.) These antibodies can be isolated from the plasma of acquired TTP patients and used *in vitro* as inhibitors of ADAMTS13. Moreover, a method of diagnosing acquired TTP is to incubate an ADAMTS13 sample with plasma from a subject to be tested for acquired TTP and to measure ADAMTS13 activity using one of the assays disclosed. Inhibition of ADAMTS13 activity by the plasma indicates the presence of inhibitory anti-ADAMTS13 antibodies in the plasma, and thus confirms a diagnosis of acquired TTP.

Klaus *et al.* (*Ibid*) performed epitope mapping experiments in order to deduce the nature of inhibitory ADAMTS13 antibodies in patients with acquired TTP. The results indicate that antibodies directed against the cysteine rich/spacer domain of ADAMTS13 were detected in all cases. In addition, antibodies directed against the TSP1-1 repeat or a fragment containing the TSP1-1 repeat, the disintegrin-like domain and the catalytic domain were detected in 72% of patients. Further, antibodies directed against the CUB 1 and/or CUB 2 domains were detected in 64% of patients. For purposes of this invention, the C-terminus consists of thrombospondin type 1 TSP1 repeats 2-8 and the CUB domains. Figure 1 shows a schematic diagram of ADAMTS13.

There is also disclosed a method for identifying inhibitors of ADAMTS13. The inhibitor can be, *inter alia,* a polypeptide, a peptide, an oligonucleotide, a. polynucleotide, a nucleoside or nucleoside analog, a saccharide, a small molecule, or a natural or synthetic chemical. The method comprises incubating a sample of ADAMTS13 with a candidate inhibitor and measuring ADAMTS13 activity according to one or more of the assays disclosed. The inhibitor is identified by reduced ADAMTS13 activity in the test sample, compared with ADAMTS13 activity in a control sample not incubated with the candidate inhibitor. The candidate inhibitor can be generated using known techniques, and can be derived, for example, from a chemical compound library, a phage display library, a natural chemical library or a combinatorial chemistry library.

### ELISA

The Enzyme Linked Immunosorbent Assay (ELISA) is a solid-phase immunoassay that is widely used in both clinical and basic research settings. In one type of ELISA, an antigen is attached to the solid phase, which is most commonly a membrane, plate, microwell or bead. The solid phase is then incubated with an antibody to the antigen (a "primary antibody"). If the primary antibody is conjugated to a label, it can be detected. This process is known as direct immunolabelling. Alternatively, the primary antibody may be unlabeled, in which case an antibody to the primary antibody (a "secondary antibody"), raised in a different species from the primary antibody and conjugated to a label, is incubated with the solid phase. This detection method is referred to as indirect immunolabelling. Immunolabelling methods are well known in the art.

In a different type of ELISA, an antibody is attached to the solid phase. This antibody can then be used to capture an antigen of interest. Direct or indirect immunolabelling techniques can be employed for the sake of analysis. In the instant invention, ADAMTS13 in a sample can be measured by binding an anti-ADAMTS13 antibody to a solid phase and adding the sample to the solid phase. ADAMTS13 present in the sample binds to the antibody, and bound ADAMTS13 is detected using direct or indirect immunolabelling. The amount of ADAMTS13 in the sample can be measured by quantifying the label.

In a preferred embodiment, the sample is or comprises platelets. In another preferred embodiment, the sample is plasma.

Another ELISA is used to detect anti-ADAMTS13 antibodies in a test sample. In this embodiment, an anti-ADAMTS13 antibody raised in a first species, *e.g*. a goat, is bound to a solid phase. ADAMTS13 is added and binds to the antibody raised in the first species. A test sample from a second species, *e.g*. human, is added, and any anti-ADAMTS13 antibodies present in the test sample bind to the ADAMTS13. Finally, a labeled antibody against antibodies from the second species is added. The labeled antibody is raised in a third species, *e.g*. donkey, and binds to the antibody from the second species. By detecting the label, one can detect anti-ADAMTS13 antibodies in the test sample.

Another ELISA provided by the invention is used to detect ADAMTS13-FXI complexes in a sample. In one embodiment, an anti-ADAMTS13 antibody is bound to a solid phase. A sample is added to the solid phase and the ADAMTS13-FXI complexes present in the sample bind to the antibody. An anti-FXI antibody is added and the ADAMTS13-FXI complex is detected by direct or indirect labeling with a chromogenic or fluorescent moiety, as described herein. The amounts of ADAMTS13-FXI complexes are quantified by detection of the label. In another embodiment, FXI is bound to a solid phase and a test sample containing ADAMTS13 is added to the solid phase and forms a complex with the immobilized FXI. An anti-ADAMTS13 antibody is added and binds to the ADAMTS13/FXI complexes. The ADAMTS13 antibody can be detected by direct or indirect labeling as described herein.

In order to detect ADAMTS13/FXI complexes using anti-ADAMTS13 antibodies, the antibodies should be reactive with the form of ADAMTS13 present in the complex. The antibodies used in the invention preferably bind to a region of ADAMTS13 within SEQ ID NO: 5. Particularly effective antibodies are the monoclonal antibodies of the invention, MAbs 4, 5, 6, 7 and 12, which were raised in mice against recombinant ADAMTS13 and screened for reactivity (*i.e.* binding) with ADAMTS13/FXI complexes. Preferred polyclonal anti-ADAMTS13 antibodies are raised in goat and rabbit. Preferred anti-ADAMTS13 antibodies recognize epitopes in the CUB 1 domain or in the domain comprising the TSP5-7 regions of ADAMTS13.

An "epitope" or "antigenic determinant" is a region on the surface of an antigenic molecule that stimulates an immune response. The epitope is the site on the antigenic molecule to which antibodies specific for the epitope bind. Epitopes preferably comprise at least 8 or 9 amino acids. Epitopes of 12, 13, 15, 18, 20 or 25 amino acids may also be identified. Methods of identifying epitopes are known in the art. Procedures such as generating overlapping peptide libraries, epitope mapping, Pepscan, electronically available bioinformatics tools, and X-ray crystallography can be used in the practice of the invention, without undue experimentation. See, *e.g*., Peters et al., Immunogenetics 57:326-36 (2005); De Groot et al., Novartis Found Symp. 254:57-72 (2003); Reijonen et al., Methods 29:282-88 (2003); De Groot et al. (1999) Nat. Biotechnol. 17:533-34; Hemmer et al. J. Pept. Res. 52(5):338-45 (1998); Geysen et al. Southeast Asian J Trop Med Public Health 21:523-33 (1990); Van der Zee et al. Eur. J. Immunol. 19:43-47 (1989); Geysen et al., Proc. Nat'l Acad. Sci. USA 82:178-82 (1985); Geysen et al., Proc. Nat'l Acad. Sci. USA 81:3998-4002 (1984). Other documents cited and incorporated herein may also be consulted for methods for determining epitopes of an immunogen or antigen and thus nucleic acid molecules that encode such epitopes.

The test sample can be any biological fluid, such as blood, platelets, plasma, serum, culture fluid, cerebral spinal fluid or sputum. In a preferred embodiment, the biological fluid is plasma.

The control sample or "normal sample" should be the same biological fluid as the test sample, collected from an individual without a hemostatic or thrombotic condition. In a preferred embodiment, the control sample is plasma from an individual having no hemostatic or thrombotic conditions. Alternatively, where appropriate, the control sample can be a preparation of isolated ADAMTS13/FXI complexes, for example, from an immunoprecipitation procedure.

The label can be any moiety known in the art, including chromogenic enzyme systems, such as horseradish peroxidase, alkaline phosphatase, β-galactosidase, glucose 6-phosphate dehydrogenase. These enzyme labels are reacted with a chromogenic substrate that can be detected by conventional techniques. In a preferred embodiment, the label is horse radish peroxidase, and the substrate is tetramethylbenzidine. The label can also be a fluorescent dye, including rhodamine, fluorescein, Cy dyes, Texas Red, and derivatives thereof.

### Diagnostic Applications

In addition to testing the inhibitory properties of plasma on an ADAMTS13 sample, the invention provides methods for diagnosing pulmonary embolism "Hemostatic disorder" and "hemostatic condition" and "abnormal hemostatic condition" are used interchangeably to mean a disorder of blood clot formation. "Hemostatic disorders" and the like include "thrombosis" and "thrombotic condition" and "thrombotic disease," which are used interchangeably to mean a disorder characterized by formation of a clot or obstruction inside a blood vessel, which obstructs normal blood flow. Therefore, diagnosis of hemostatic disorders or conditions includes thrombotic conditions as well.

Thrombosis or thrombophilia may be diagnosed generally, without regard to the underlying cause. Examples of particular thromobotic or hemostatic conditions that can be detected include, but are not limited to, TIP, hemophilia A, B and C, deep venous thrombosis (DVT), portal vein thrombosis, renal vein thrombosis, hepatic vein thrombosis (Budd-Chiari syndrome), Paget-Schroetter disease, thoracic outlet syndrome, disseminated intravascular coagulation (DIC), heparin induced thrombocytopenia (HIT), antiphospholipid syndrome (APS), and lupus anticoagulant (LA). Thrombotic conditions may be venous or arterial. Thromboses may be caused by an underlying condition, such as sickle cell anemia, pulmonary hypertension, ischemic stroke, myocardial infarction, rupture of atherosclerotic plaque or cancer.

Diagnosis of TTP, not forming part of the present invention, is used as an example in the following discussion; however, the diagnostic methods are equally applicable to other hemostatic or thrombotic conditions. Congenital or acquired TTP can be diagnosed by measuring ADAMTS13 activity in a test sample from a subject and subsequently comparing the ADAMTS13 activity in the test sample to ADAMTS13 activity in a control sample having normal ADAMTS13 activity. Reduced ADAMTS13 activity in the test sample compared with the control sample indicates a positive diagnosis of TTP.

Congenital or acquired TTP can also be diagnosed by measuring the amount of ADAMTS13/FXI complexes in a test sample from a subject using the ELISA described herein and subsequently comparing the amount of complexes in the test sample to the amount of complexes in a control sample having normal ADAMTS13 activity. A level of ADAMTS13/FXI complexes in the test sample different from the control sample indicates a thrombotic or abnormal hemostatic condition. In the-case of TTP, the level of complexes is elevated in TTP patients as compared with samples from normal patients.

PE is generally diagnosed clinically, as there is no reliable laboratory test to diagnose PE. The most common laboratory test performed on patients with suspected PE is a D-dimer assay; however, this assay is useful only in a negative context to rule out PE. On the other hand, in accordance with the present invention, elevated ADAMTS13/FXI complexes indicate PE. The assay of the invention is particularly useful in a differential diagnosis context, whereby it can determine whether a patient with DVT has PE as well.

The assays disclosed may detect the presence of a hemostatic disorder in an individual prior to the time that such a disorder could be detected clinically. Therefore, the invention is advantageous because it provides an initial indication that a hemostatic disorder exists in the individual. Further diagnostic testing may be desirable to determine the particular nature of the hemostatic disorder.

Statistical significance can be measured by any known technique, including a student's t-test, a chi-square test, an analysis of variance (ANOVA), Dunn's multiple comparison test, and the like.

### Treatment Applications

The techniques provided can be used to monitor treatment of a patient for hemostatic or thrombotic disorders. For example, during treatment, ADAMTS13 activity can be measured using the assays disclosed. This allows the clinician to assess the efficacy of the treatment and/or to determine the length of a treatment session that is required to restore ADAMTS13 activity.

One treatment for TTP is plasmaphoresis. In patients with acquired TTP, plasmaphoresis is used to remove the inhibitory anti-ADAMTS13 antibodies from the patient's plasma. Replacement of the deficient ADAMTS13 is provided by infused plasma. Recent advances in our understanding of the pathological mechanisms of TTP provide a rationale for monitoring plasmaphoreseis via measuring the levels of ADAMTS13 activity in the patient undergoing plasmaphoresis.

There is also disclosed a method for treating TTP in a patient in need thereof comprising administering to the patient a drug that specifically targets platelet ADAMTS13. The drug can be identified using the screening methods provided by the invention and described herein. The term "drug" is meant to encompass a polypeptide, a peptide, an oligonucleotide, a polynucleotide or vector containing a polynucleotide, a nucleoside or nucleoside analog, a saccharide, a small molecule, or a natural or synthetic chemical.

The invention describes the interaction of ADAMTS13 with FXI and FXIa and the ability of ADAMTS13 or a fragment thereof to form a stable complex with FXI and FXIa. The invention provides methods to identify and detect a ADAMTS13/FXI complex. It is possible that administering a drug that interferes with the interaction of ADAMTS13 and FXI /FXIa may be useful for treating patients with hemostatic disorders. The drug can be identified using the screening methods provided by the invention whereby the drug interferes with the binding of ADAMTS13 to FXI as described herein.

The invention will now be further described by way of the following non-limiting Examples, given by way of illustration.

### EXAMPLES

### Materials and Methods

### Synthetic Peptidyl Substrates

Leu-Val-Tyr-AMC (LVY-AMC) and Suc-Leu-Leu-Val-Tyr-AMC (Suc-LLVY-AMC) (SEQ ID NO: 2) were obtained from Bachem Bioscience Inc. (King of Prussia, PA). LVY-AMC was also made by QPR (Montreal, Canada). FRET substrate, NH₂-Arg-Lys(*DABCYL)-*NLVYMVTGD(EDANS)-Arg-COOH (SEQ ID NO: 1), was made for American Diagnostica Inc. by Molecular Biology Resource Center, University of Oklahoma Health Sciences Center (Oklahoma City, OK).

### Preparation of Platelet Rich Plasma (PRP), Platelet Poor Plasma (PPP) and Isolated Platelets

Blood was collected by venopuncture into collection tubes containing 0.12 M sodium citrate, an anti-coagulant. The citrated blood was centrifuged at 700 rpm for 10 minutes. The supernatant was removed and saved as the platelet rich plasma (PRP) fraction. The PRP was centrifuged at 10,000 rpm for 20 minutes. The supernatant was removed and saved as the platelet poor plasma (PPP) fraction. The pellet contained the platelets, which were washed by resuspending in 10 mM Tris-HCl pH 8.0 buffer and centrifuging at 10,000 rpm for 10 minutes. The isolated platelets were resuspended in buffer and used in experiments.

### Recombinant ADAMTS13

HEK 293 cells were transfected with a vector encoding ADAMTS13. Cell culture supernatant from the transfected HEK 293 cells, containing recombinant ADAMTS13, was generously provided by Dr. David Ginsburg (University of Michigan, MI). Mock transfected cell supernatant with no recombinant ADAMTS13 was also provided as a control.

### Example 1. Measurement of ADAMTS13 Activity in PRP Using LVY-pNA and LVY-AMC Peptidyl Substrates

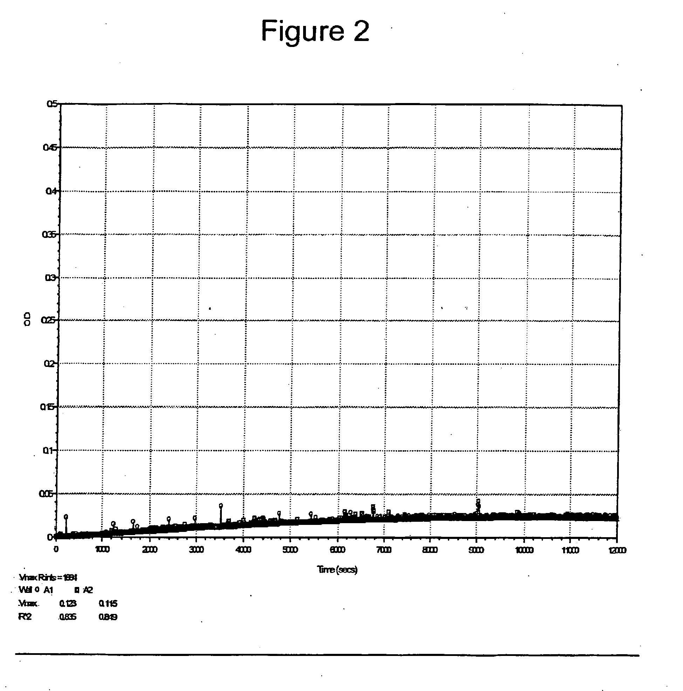

As discussed above, Furlan *et al.* reported that some peptidyl-pNA moieties, such as XLY-pNA and XVY-pNA, were not substrates for ADAMTS13. We prepared LVY-pNA and tested it for its ability to be cleaved by ADAMTS13. The results shown in Figure 2 confirm the findings of Furlan *et al*., *i.e*., LVY-pNA was not cleaved by ADAMTS13.

Thus, the nature of the leaving group of the peptidyl substrate appears to be important in determining the ability of ADAMTS13 to cleave a chromophor or a fluorophor from the end of a peptidyl substrate.

### Example 2. Measurement of ADAMTS13 Activity in PRP Using Peptidyl Substrates of Different Lengths

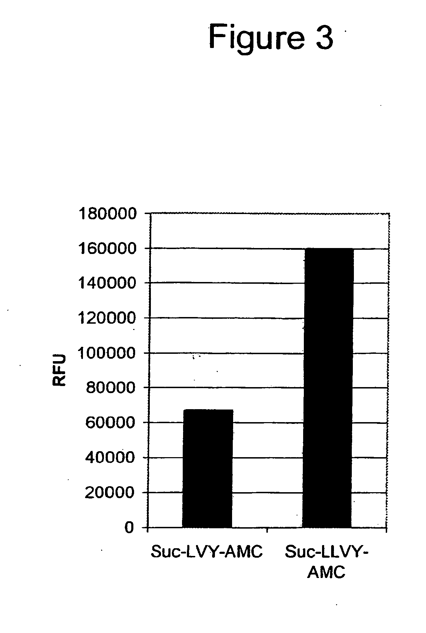

In order to determine the effect of modifying the length of the peptide sequence conjugated to the AMC fluorochrome, normal PRP (20 µl) was incubated at 37° C with a final concentration of 0.8 mM Suc-LLVY-AMC or LVY-AMC in 10 mM Tris-HCl pH 8.0 buffer in total volume of 200 µl. Fluorescence was measured using a microtiter fluorophotometric plate reader (Ex 360 nm/ Em 460 nm). Both peptidyl-AMC substrates were cleaved by ADAMTS13 in PRP, with the Suc-LLVY-AMC giving a higher signal over time as compared to the LVY-AMC substrate (Figure 3).

This example demonstrates that different ADAMTS13 substrates can be made by altering the peptide sequence that is conjugated to the AMC fluorophor.

### Example 3. Measurement of ADAMTS13 Activity Using a FRET Substrate

ADAMTS13 activity was measured using a fluorescent substrate attached to a donor moiety and an acceptor moiety that functions by fluorescence resonance energy transfer (FRET). The ADAMTS13 selective substrate, NH₂-Arg-Lys(*DABCYL)*-NLVYMVTGD(*EDANS)-*Arg-COOH, was used in this example. The NLVYMVTGD peptide sequence of this substrate is homologous to the ADAMTS13 cleavage site on VWF. The intact peptidyl substrate has low fluorescence, due to quenching of the DABCYL-fluorochrome by the EDANS-quencher. Cleavage of the substrate between the tyrosine and methionine of the peptide sequence results in an increase in fluorescence.

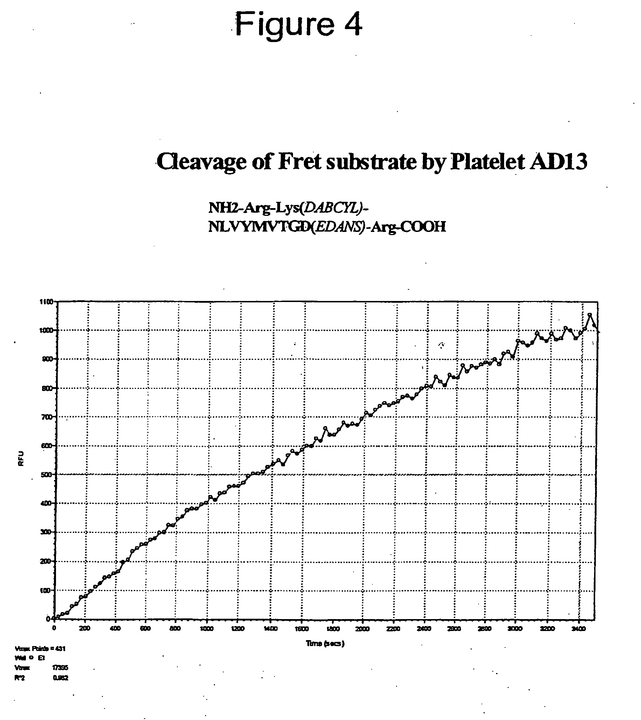

Isolated platelets in 10mM Tris-HCl pH 8.0 assay buffer were incubated with a final concentration of 8 µg/ml of the FRET substrate at 37°C. The fluorescence was measured in a spectrofluorometric plate reader (Ex 360 nm/ Em 440 nm). Figure 4 shows that incubation of platelets with NH₂-Arg-Lys(*DABCYL)*-NLVYMVTGD(*EDANS)*-Arg-COOH results in increased fluorescence over time, indicating that the form of ADAMTS13 found on platelets cleaves this FRET peptide substrate. The skilled artisan can design other FRET substrates for measuring ADAMTS13 activity using his or her own knowledge and the teachings herein.

### Example 4. Measurement of Recombinant ADAMTS13 Activity in Tissue Culture Supernatants Using Suc-LLVY-AMC Fluorescent Substrate

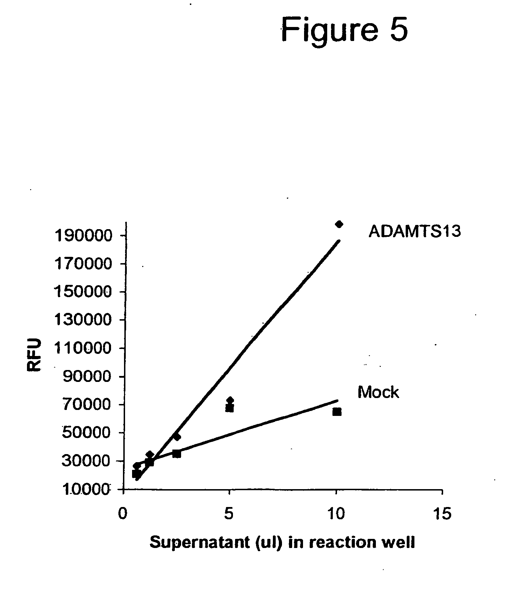

Fluorescence signals were compared between tissue culture supernatant from HEK 293 cells transfected with a viral vector coding for recombinant ADAMTS13 ("recADAMTS13") and supernatant from mock transfected HEK 293 cells, as a control. (Culture supernatants were supplied by Dr. David Ginsburg, University of Michigan.) Varying amounts of the two culture supernatants were added to 0.8 mM Suc-Leu-Leu-Val-Tyr-AMC (SEQ ID NO: 2) fluorescent substrate in 50 mM Tris-HCl pH 8.0 in a total volume of 100 µl. Figure 5 shows that a greater fluorescence signal was produced by the supernatant from transfected cells verses supernatant from mock transfected cells. The increased fluorescence signal is due to cleavage of the substrate by recADAMTS13 in supernatant of the vector transfected cell supernatant.

### Example 5. Measurement of ADAMTS13 Activity in Plasma

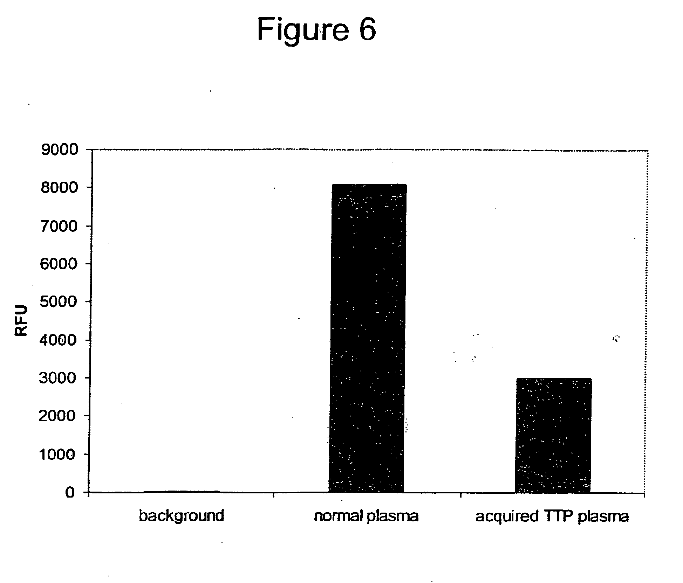

ADAMTS13 activity was measured in plasma using the Suc-LLVY-AMC substrate. Normal plasma (50 µl) or acquired TTP plasma (50 µl) was added to substrate in 50 mM Tris-HCl pH 8.0 buffer. The final concentration of the substrate was 0.8 mM; substrate in buffer was used as a background control. The reaction mixture was incubated at 37° C for 4 hours and fluorescence was recorded in a spectrofluorometric plate reader at Ex 360 nM/ Em 440 nM (Figure 6). Higher ADAMTS13 activity was observed in the normal plasma as compared to acquired TTP plasma.

### Example 6. Comparison of ADAMTS13 Activity in PRP from Normal and TTP Subjects

The ADAMTS13 activity from normal plasma and from acquired TTP plasma were compared in the fluorescence assay described above. Specifically, 20 µl of normal PRP were serially diluted 1:2 in normal PPP in microtiter wells. Eighty µl of LVY-AMC (0.2 mM final concentration) in 10 mM Tris-HCl pH 8.0 buffer were added to the PRP. The microtiter plate was placed in a spectrofluorometric microtiter plate reader at 37° C and the fluorescence was monitored at Ex 360 nm/ Em 440 nm for 16 minutes.

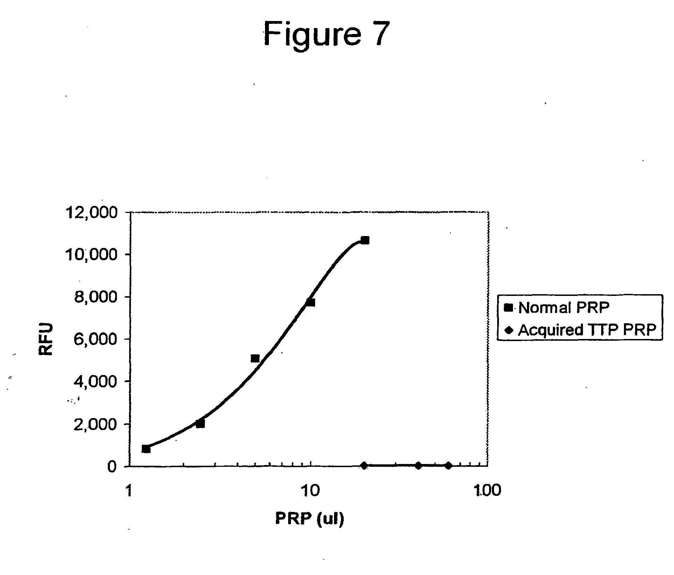

The results shown in Figure 7 demonstrate that, as the amount of PRP in the assay decreased, there was a decrease in the fluorescence signal. PRP from a patient diagnosed with acquired TTP was-tested in the assay under the same conditions as normal PRP. In this experiment, more plasma from the acquired TTP patient (20-60 µl) was used in the assay, as compared to normal PRP. No fluorescent signal was generated at the highest amount of PRP from the acquired TTP patient. This shows that ADAMTS13 activity in PRP from an acquired TTP patient has significantly less activity than normal PRP. This method can be used to diagnose deficiency in ADAMTS13 activity.

### Example 7. ADAMTS13 Activity Is Associated with Platelets

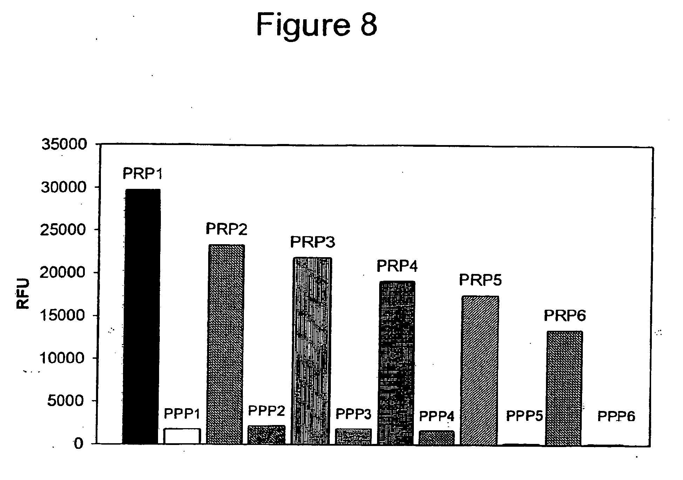

PRP and PPP from six normal volunteers were prepared as described above. Fifty µl of each plasma sample were added to 130 µl 50 mM Tris-HCl buffer pH 8.0 and 20 µl of 8 mM Suc-LLVY-AMC substrate. The reaction mixtures were incubated at 37° C and monitored for 1 hour in a spectrofluormetric plate reader at Ex 360 nm/ Em 440 nm. The six PRP samples exhibited high ADAMTS13 activity, as evidenced by generation of a high fluorescence signal (RFU) over time (Figure 8).

The rate of fluorescence generation over time was significantly greater in PRP than in plasma. After pelleting of platelets, the PPP from each subject was also tested, and exhibited very little ADAMTS13 activity. These results show that the majority of ADAMTS13 activity in PRP is present on the platelets and not in the plasma.

### Example 8. Reduced ADAMTS13 Activity is Associated with Human Platelets from an Acquired TTP Patient

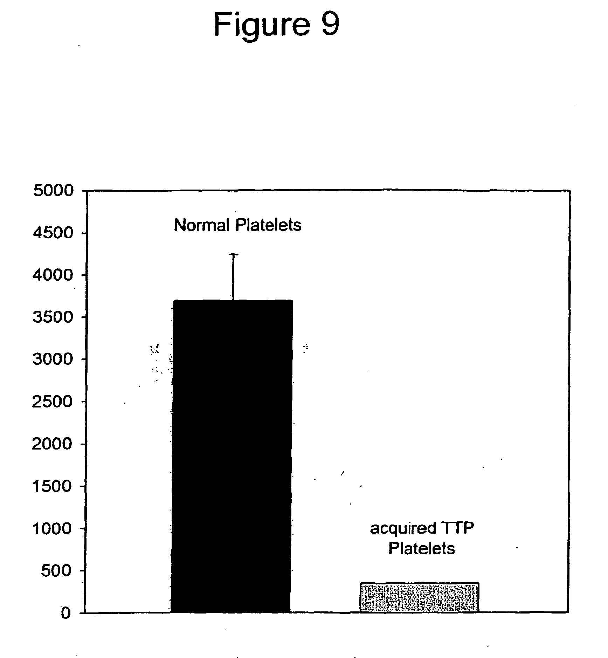

Isolated platelets from a normal subject and from a patient diagnosed with acquired TTP were prepared as described above. Approximately 600 µg of protein in 100 µl of 50 mM Tris-HCl pH 8.0 buffer from the normal and the acquired isolated platelets were placed in wells in a fluorescence microtiter plate. LVY-AMC substrate was added to each tube to a final concentration of 0.2 mM and the reaction mixtures were incubated at 37° C for 1 hour. The fluorescence was monitored at Ex 360 nm/ Em 440 nm. The amount of ADAMTS13 activity per mg of protein associated with normal platelets was significantly greater than that associated with platelets from a patient with acquired TTP (Figure 9).

This method can be used to quantitate ADAMTS13 activity on platelets. Low ADAMTS13 activity would indicate TTP. Both congenital TTP, caused by mutation or alteration of ADAMTS13, and acquired TTP, caused by presence of ADAMTS13 inhibitory antibodies, can be diagnosed using this method.

### Example 9. ADAMTS13 Associated with Platelets is Present in a Cleaved Form

The molecular form of ADAMTS13 in platelets was investigated by immunostaining of Western blots of platelet proteins (Figure 10). Washed platelets were solubilized in SDS-sample buffer with and without mercaptoethanol. SDS-PAGE electrophoresis was performed using 4-20% acrylamide gel. The resolved proteins were electroblotted onto nylon paper and immunostained with different biotinylated anti-ADAMTS13 antibodies and strepavidin-HRP/TMB. A goat anti-ADAMTS13 antibody specifically immunostained protein bands under reducing conditions at approximately 120 kD, 43 kD and 30 kD. Using non-reducing conditions, a high molecular weight band at approximately 150 kD was specifically immunostained.

These findings suggest that ADAMTS13 on platelets is comprised of more than one protein held together by disulfide bonds. ADAMTS13 in plasma has been reported to be comprised of a single protein of molecular weight 150-170 kD. The ADAMTS13 associated with platelets is different than plasma ADAMTS13, and appears to be cleaved. ADAMTS13 from platelets was immunostained using plasma from an acquired TTP patient. A high molecular weight band was specifically immunostained under non-reducing conditions. Reduction of the protein caused the ADAMTS13 not to be stained by this TTP plasma.

### Example 10. Enhanced Activity of Platelet ADAMTS13 by Treatment with a Proteolytic Enzyme

ADAMTS13 on platelets appears to be proteolytically cleaved into at least two or more polypeptide chains held together by disulfide bonds, whereas plamsa ADAMTS13 is a single peptide chain. We tested whether a peptidase can cleave ADAMTS13 and increase activity toward ADAMTS13 peptide substrates.

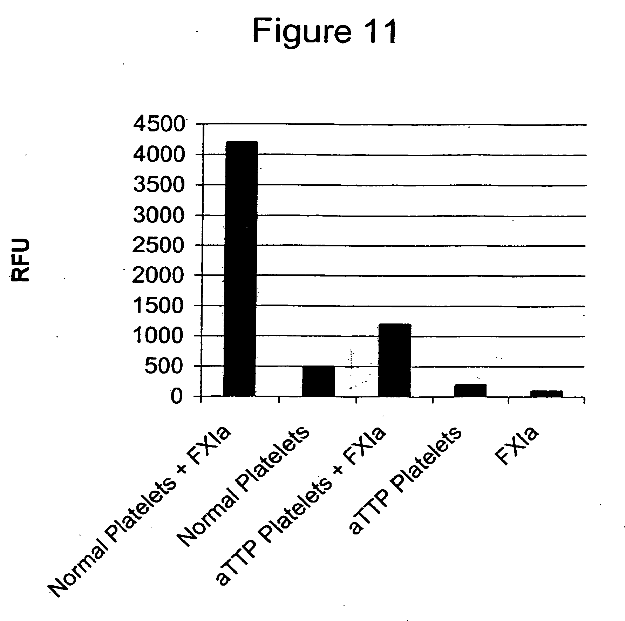

Figure 11 shows that ADAMTS13 activity on platelets is enhanced by treatment with peptidase activated FXIa. Washed normal platelets from 200 µL of PRP were resuspended in 1 ml of assay buffer. Washed platelets from 1 ml of PRP from a subject with acquired TTP were resuspended in 50 µL of assay buffer. Normal or TTP platelets (10 µl) were added to 10 mM Tris-HCl pH 8.0 assay buffer (80 µl) or assay buffer containing 0.3 ng/ml of FXIa (80 µl). LVY- AMC substrate (10 µl of 2 mM) was added and the fluorescence was monitored (Ex 360 nm/ Em 440 nm) for 1500 seconds. FXIa at 0.3 ng/ml was used as a control. ADAMTS13 activity was significantly increased (by approximately 8-fold) towards LVY-AMC substrate upon treatment of platelets with FXIa. Activity of platelets isolated from PRP of an acquired TTP patient was enhanced by treatment with FXIa to a much lesser extent than those isolated from PRP of a normal individual. This shows that the inhibitory antibodies in TTP plasma block activation of platelet ADAMTS13 by FXIa.

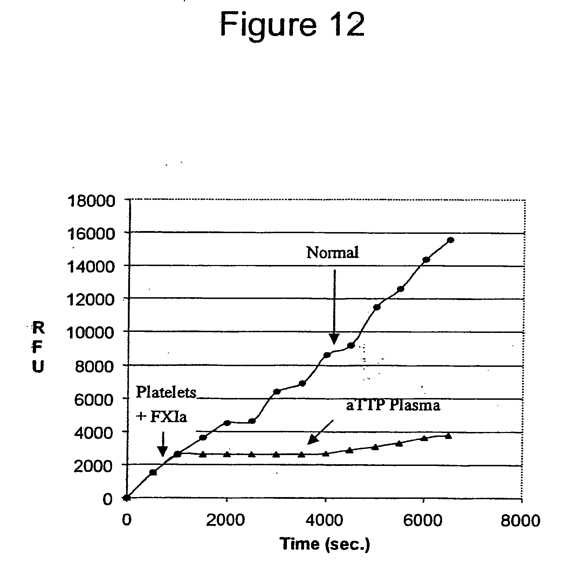

The activity of FXIa treated platelets towards LVY-AMC was significantly reduced after addition at 1500 seconds of 50 µl acquired TTP plasma to the reaction mixture, whereas, addition of 50 µl normal plasma had no significant effect on the activity (Figure 12). These results show that the enhancement of platlelet ADAMTS13 activity by FXIa is blocked by anti-ADAMTS13 antibodies. These findings also show that ADAMTS13 activity can be enhanced by treatment with proteolytic enzymes.

These findings suggest that ADAMTS13 present on platelets is in a different form than that found in plasma. Results above show that platelet ADAMTS13 appears to have greater enzymatic activity towards peptidyl substrates than plasma ADAMTS13. The finding of proteolytically cleaved ADAMTS13 on platelets may explain the differences in reactivity of plasma and platelet-bound ADAMTS13 towards peptidyl substrates.

### Example 11. Measuring ADAMTS13 Antigen in Plasma Using ELISA

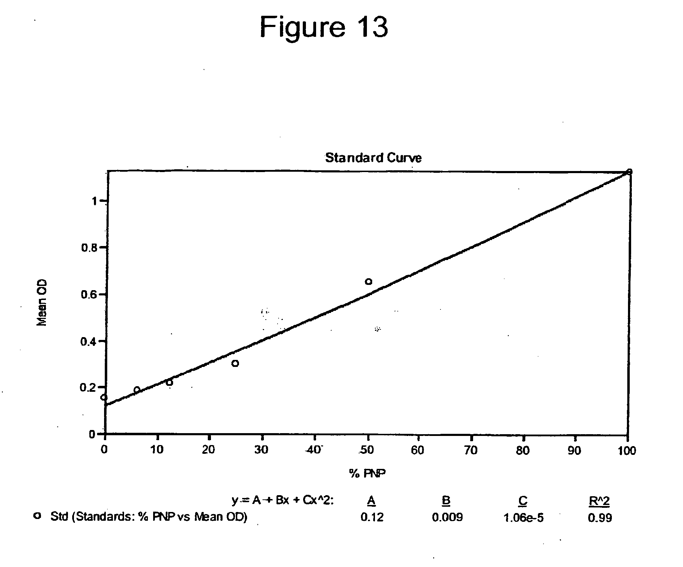

An ELISA for measuring ADAMTS13 protein in biological fluids was developed. Immulon 4 96-well microtiter plates (Dynex) were coated with goat anti-ADAMTS13 antibody (2 µg/ml) in 100 µl of 50 mM MOPS buffer pH 6.0. Plates were washed and blocked with Superblock (Pierce, IN). A PNP sample was serially diluted 1:2 in buffer and 100 µl was added to microtiter wells. After incubation for 1 hour at 37°C, the plate was washed and 0.5 µg/ml immunoglobulin purified from plasma obtained from a patient with acquired TTP was added to the microtiter wells. After incubation at 37° C for 1 hour, the plate was washed and donkey anti-human Ig-HRP labeled antibody (Jackson Laboratories, ME) (1:1000) was added to the wells. After incubation for 1 hour at 37° C, the plate was washed and 100 µl TMB substrate (Moss Inc, MD) was added to each well. The plate was incubated at room temperature for 5 minutes and the reaction was stopped by adding 50 µl of 0.45 M sulfuric acid. The absorbance at 450 nm was measured. Figure 13 shows the increase in absorbance was linearly proportional to the amount of ADAMTS13-containing plasma added.

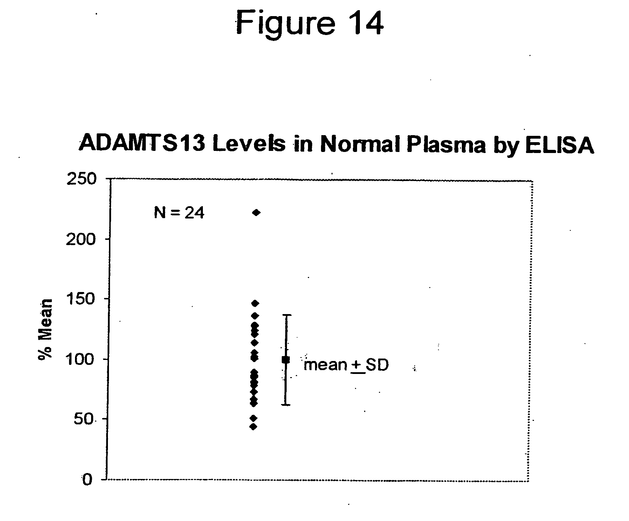

In another experiment, the amount of ADAMTS13 protein in 24 normal plasma samples was determined using the ELISA method. The results are shown in Figure 14. Using PNP as 100%, the amount of ADAMTS13 in 24 individual normal plasma samples was distributed around the PNP value. This ELISA method can be used to determine the amount of ADAMTS13 in plasma and other biological fluids.

### Example 12. Inhibition of Recombinant ADAMTS13 Activity by Goat anti-ADAMTS13 Antibody

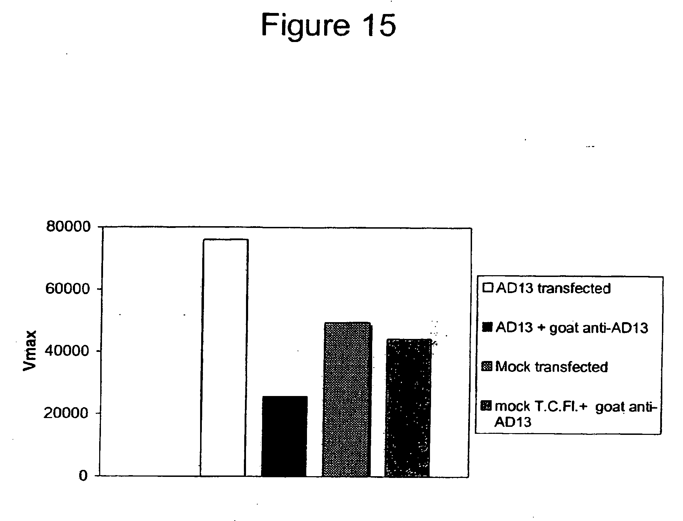

Tissue culture fluid (5 µl) from HEK 293 cells transfected with a vector coding for recADAMTS13 or tissue culture fluid (5 µl) from mock transfected HEK 293 cells was added to 5 µg of goat anti-ADAMTS13 antibody (Santa Cruz Biochemicals, Santa Cruz, CA) and 0.8 mM Suc-LLVY-AMC fluorescent substrate in 50 mM Tris-HCl pH 8.0 buffer (85 µl). The fluorescence (Vmax) was followed in a spectrofluorometric plate reader at Ex 360 nm/ Em 440 nm (Figure 15).

ADAMTS13 culture fluid had more fluorescence activity than the mock transfected culture fluid. The goat anti-ADAMTS13 antibody inhibited the fluorescence from the ADAMTS13 fluid but not from the tissue culture fluid from mock transfected cells. These results show that the fluorescence activity from the fluid from mock transfected cells is not due to ADAMTS13.

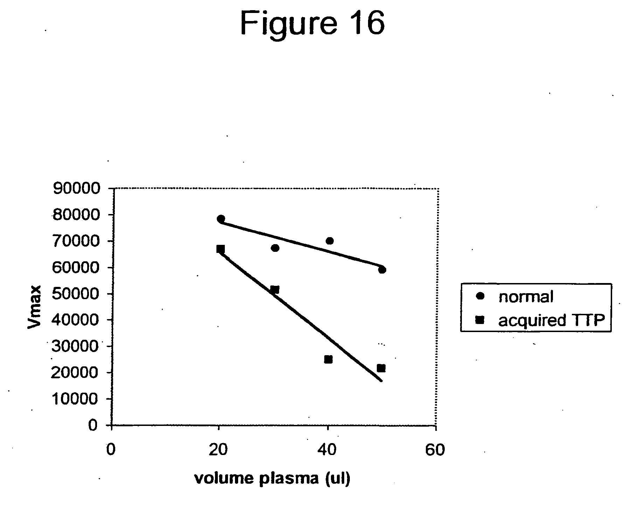

Example 13. Inhibition of Recombinant **ADAMTS13** Activity by Acquired TTP Plasma Plasma from patients with acquired TTP ("acquired TTP plasma") contains an inhibitory antibody against ADAMTS13. Figure 16 shows that the addition of TTP plasma to tissue culture supernatant containing recADAMTS13 inhibited the generation of a fluorescence signal when Suc-LLVY-AMC was used as a substrate. The amount of inhibition of fluorescence signal was dependent on the amount of TTP plasma added. Plasma from healthy subjects ("normal plasma") did not significantly inhibit ADAMTS13 activity, as compared to acquired TTP plasma, demonstrating the ADAMTS13 activity was specifically inhibited by the acquired TTP plasma. These studies also show that antibodies present in acquired TTP plasmas block hydrolysis of the fluorescent Suc-LLVY-AMC substrate by recADAMTS13.

### Example 14. Inhibition of ADAMTS13 Activity on Isolated Platelets by Anti-ADAMTS13 Antibodies

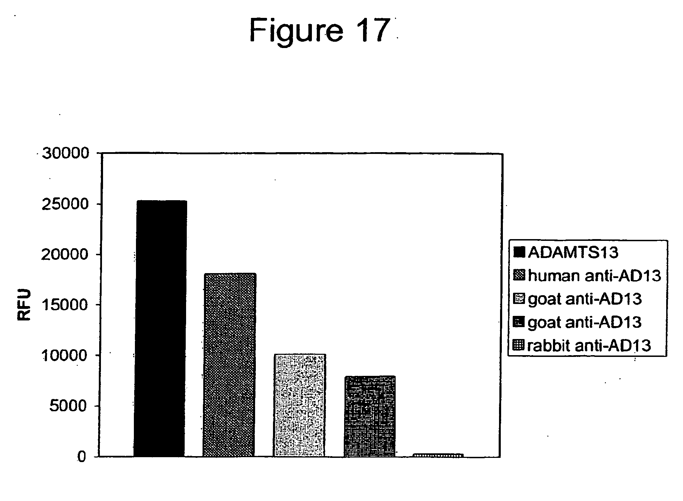

One ml of PRP was centrifuged at 10,000 rpm and the platelets in the pellet were washed with 50 mM Tris-HCl pH 8.0 buffer. The platelets were suspended in 200 µl of assay buffer. Isolated platelets (20 ul) were added to 70 µl of various anti-ADAMTS13 antibodies and incubated at room temperature for 15 minutes. The G1 and G2 goat antibodies (Santa Cruz Biochemicals, Santa Cruz, CA) were used at 200 µg/ml; the rabbit antibody against the C-terminal fragment of ADAMTS13 (from Dr. Ginsburg) was used at 5.5 mg/ml. Purified immunoglobulin (Ig) from acquired TTP plasma was used at a concentration of 4 mg/ml. Ninety µl of 10mM Tris-HCl pH 8.0 buffer and 10 µl of 8 mM LVY-AMC were added and the fluorescence was measured over time as above. All four anti-ADAMTS13 specific antibodies inhibited the activity of normal platelets (Figure 17). These findings indicate that the activity in platelets was due to ADAMTS13 cleaving the LVY-AMC substrate.

### Example 15. A Method for Measuring Anti-ADAMTS13 Antibodies in Plasma Using Isolated Platelets

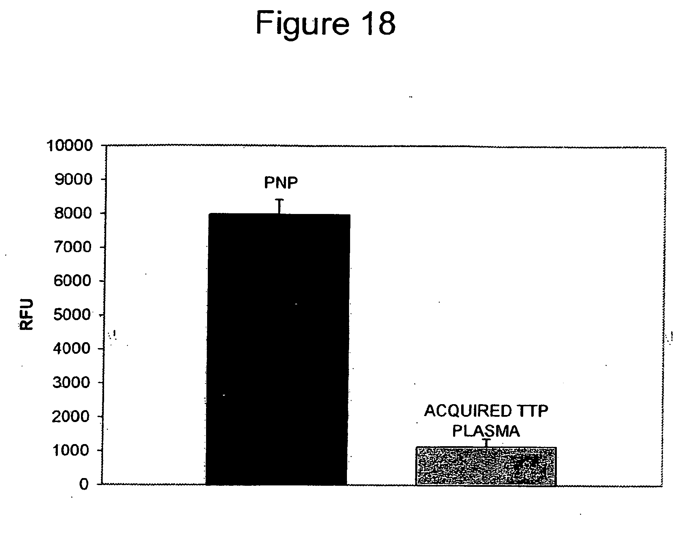

Detection of anti-ADAMTS13 autoantibodies was performed using isolated platelets as a source of ADAMTS13. Isolated platelets (prepared from 200 µL of normal PRP) were incubated with 200 µL pooled normal plasma (PNP) or acquired TTP plasma. The mixtures were incubated at 37°C for 30 minutes. Fifty µl were removed from each reaction mixture and added to 130 µl of 50 mM Tris-HCl pH 8.0 buffer and 20 µL of 8 mM LVY-AMC substrate. The reaction mixtures were incubated for 30 minutes at 37° C for one hour and fluorescence was monitored in a spectrofluorometric plate reader (Ex 360 nm/ Em 440 nm). The platelets incubated with normal plasma had high fluorescence signal (indicating high ADAMTS13 activity), whereas the platelets incubated with acquired TTP plasma had low fluorescence signal (indicating low ADAMTS13 activity) (Figure 18). These results show that anti-ADAMTS13 autoantibodies were present in the acquired TTP plasma, but not in the normal plasma, and that autoantibodies in plasma from a patient with acquired TTP can be detected using this method. Other fluorescent ADAMTS13 substrates can be substituted for LVY-AMC. This method can be used to distinguish between congenital TTP and acquired TTP.

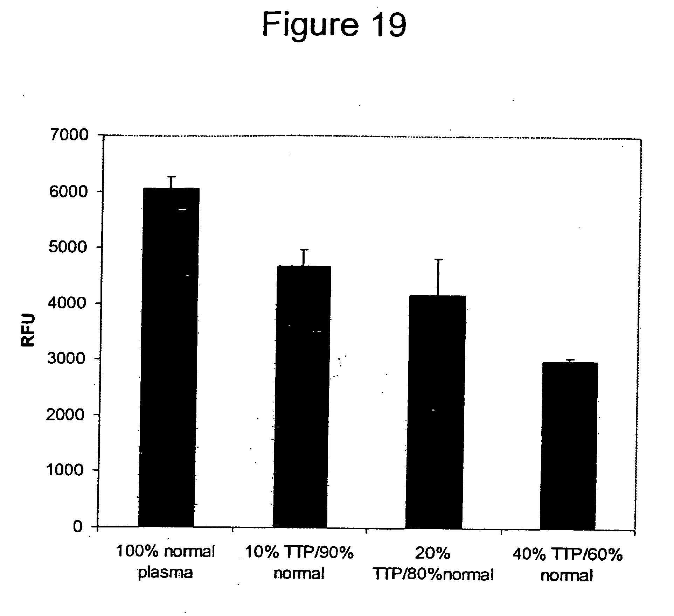

Figure 19 shows that the level of inhibition of ADAMTS13 activity on platelets is dependent on the amount of anti-ADAMTS13 autoantibodies used in the assay, and thus the anti-ADAMTS13 activity. Plasma from an acquired TTP patient was diluted to different amounts (10%, 20% and 40%) in normal plasma and incubated with isolated platelets in a total volume of 90 µl for 15 minutes. Ten µl of 8 mM LVY-AMC substrate was added and the fluorescence was determined after incubation at 37°C for 1 hour. The inhibition of ADAMTS13 activity by TTP plasma was dependent upon the amount of TTP plasma used in the assay.

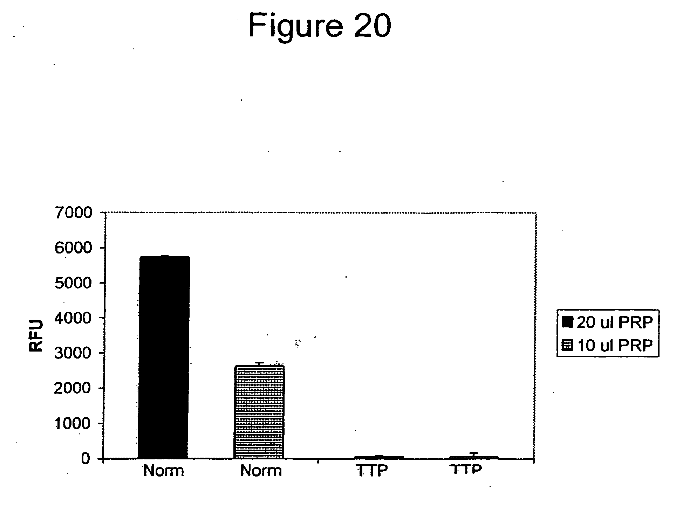

The amount of fluorescence generated over time in the assay is dependent upon the amount of platelets used in the assay. As shown in Figure 20, a higher fluorescent signal was generated in the control (normal plasma without anti-ADAMTS13 antibodies) using more platelets. The addition of acquired TTP plasma, containing ADAMTS13 antibodies, almost completely inhibited ADAMTS13 activity, regardless of the amount of platelets in the assay.

### Example 16. Detection and Measurement of ADAMTS13/FXI Complexes in Plasma

An assay was developed for measuring the presence of ADAMTS13/FXI complexes by ELISA analysis. A standard curve was generated using PNP (Saturn Biomedical), diluted 1:4 in ECD buffer (PBS/3% BSA/gentamicin), and then serially diluted 1:2 in ECD buffer. Assays were performed in duplicate, using 100 µl of each standard dilution in Immulon 4 HBX 96-well microtiter plates (ThermoLabsystems, Franklin, MA) that were coated with goat anti-human ADAMTS13 antibody raised against a synthetic peptide sequence in the CUB1 domain (SC21510; Santa Cruz Biotechnology, Santa Cruz, CA) at a concentration of 1 µg/ml in 3-(N-morpholino)propanesulfonate (MOPS) coating buffer (0.1 M MOPS, 0.15 M NaCl, 0.05 M CaCl₂, 0.066 M Trehalose, 0.02% NaN₃, pH 7.4). Microtiter plates were blocked with SuperBlock (Pierce Chemical Co., Indianapolis, IN).

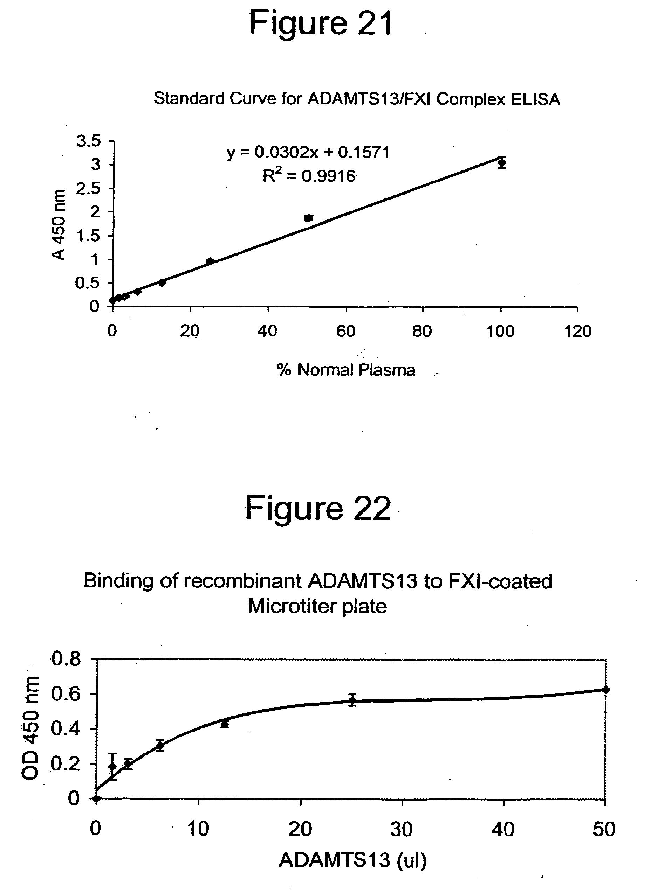

Standards and diluted samples were incubated in coated microwells for 2 hours at room temperature, with mixing. The plate was then washed four times with wash buffer (PBS + 1% Tween, pH 7.4). An anti-FXI antibody labeled with HRP was diluted to 1 µg/ml in 7% normal goat serum in ECD buffer, and then 100 µl of the diluted detection antibody was added to each well. The antibody was allowed to bind for one hour at room temperature. Following incubation with the detection antibody, the plate was washed four times in wash buffer. Tetramethylbenzidene HRP substrate (TMB-HRP; 100 µl) was then added to the microwells. After 15 minutes, the reaction was terminated with 0.5 N sulfuric acid. The optical density of the mixture was then read in a spectrophotometer at 450 nm. The standard curve generated is shown in Figure 21.

### Example 17. Binding of Recombinant ADAMTS13 to FXI-Coated Plates

Binding of recADAMTS13 to immobilized FXI was measured using microplates coated with human FXI at a concentration of 1 µg/ml. RecADAMTS13 in solution was added to the FXI-coated microwells and incubated for 1 hour. ADAMTS13/FXI complexes were measured using ELISA. Specifically, after incubation with recADAMTS13, plates were washed with PBS + 1% Tween, followed by incubation for 1 hour with anti-ADAMTS13 TSP5-7 rabbit polyclonal antibodies (1 µg/ml). Plates were washed again in PBS + 1% Tween, followed by addition of a murine anti-rabbit IgG antibody conjugated to HRP. The detection antibody was incubated for an hour at room temperature. After washing the excess detection antibody from the plates, the TMB substrate was added, incubated for 10 minutes, after which 0.5 M sulfuric acid was added to terminate the reaction. The optical density of the mixture was measured at 450 nm on a spectrophotometer (Figure 22). The results show that recADAMTS13 can form a stable complex with FXI.

### Example 18. Measurement of ADAMTS13-FXI Complexes in Acquired TTP Plasma

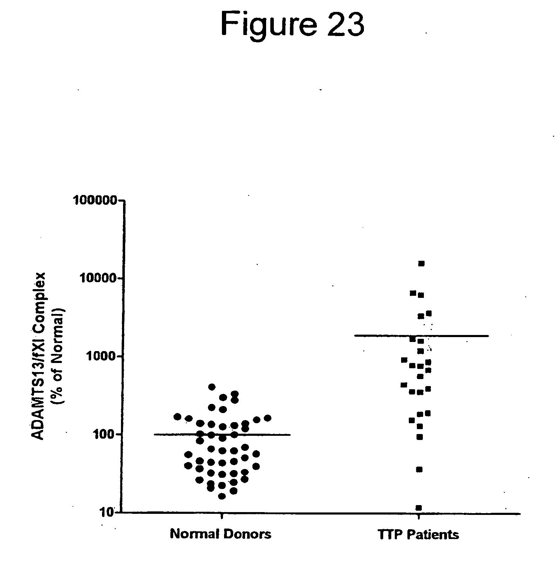

ADAMTS13/FXI complexes in plasma from 46 normal individuals and 25 patients with acquired TTP were measured as described in Example 16. Figure 23 shows the mean levels of ADAMTS13/FXI in normal plasma and TTP plasma. The data support the conclusion that ADAMTS13/FXI complex formation in acquired TTP plasma is higher than in normal plasma.

### Example 19. Formation of ADAMTS13/FXI Complexes in FXI-Deficient Plasma

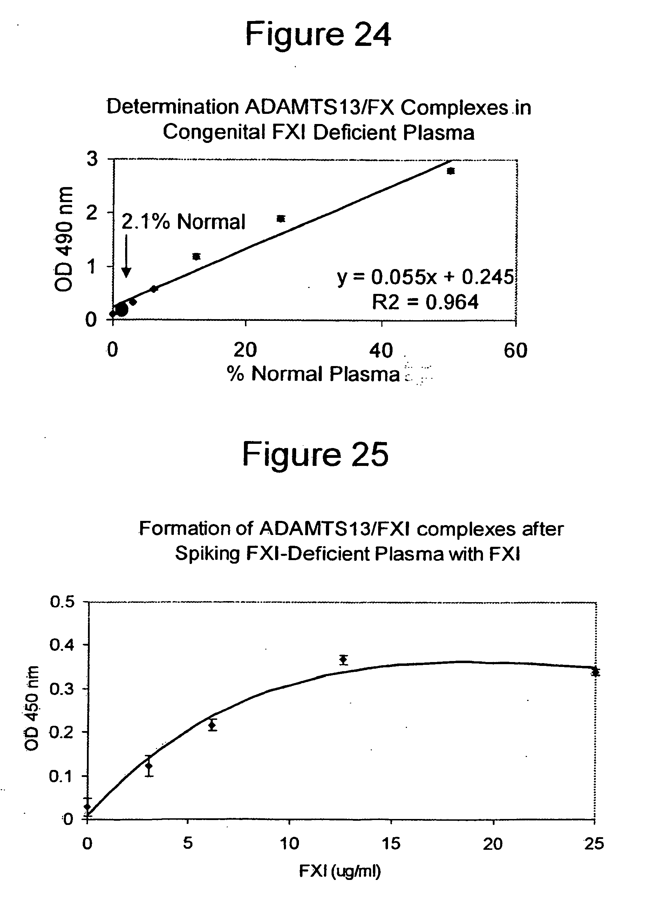

Complex formation was measured in plasma from patients with a congenital FXI deficiency using the ELISA of Example 16. The results show a significantly lower level of ADAMTS13/FXI complexes in FXI-deficient plasma than in normal plasma. As Figure 24 shows, the amount of complexes in FXI-deficient plasma are approximately equivalent to 2.1% of the amount in normal plasma.

Formation of ADAMTS13/FXI complexes was measured after addition of FXI (Enzyme Research Lab, Canada) to FXI-deficient plasma diluted 1:8 in ECD. Differing amounts of FXI were added to FXI-deficient plasma incubated for 30 minutes to promote complex formation. The plasma containing newly formed ADAMTS13/FXI complexes were then added to anti-ADAMTS13 antibody-coated microwells and quantified, as described in Example 16. Figure 25 shows that increasing amounts of ADAMTS13/FXI complexes were formed upon addition of increasing amounts of FXI to the FXI-deficient plasma.

### Example 20. Measurement of ADAMTS13/FXI Complexes in Patients Diagnosed with a Thrombotic Condition

Citrated frozen plasma samples were obtained from patients diagnosed pulmonary embolism (PE). Plasmas were assayed for ADAMTS13/FXI complexes using the ELISA method.

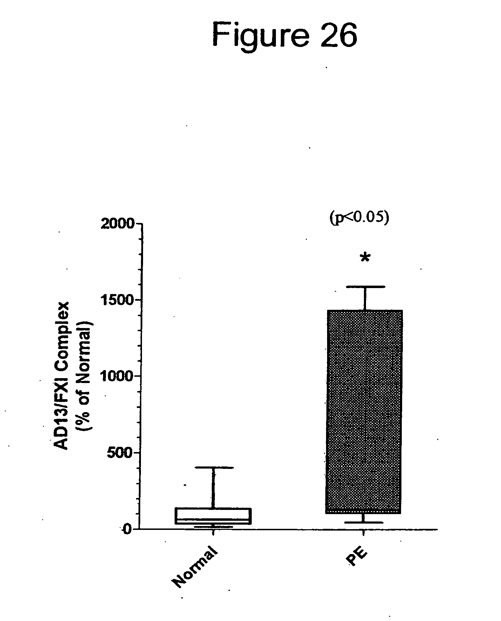

In one experiment, the ELISA was performed using a goat anti-ADAMTS13 polyclonal antibody directed against the CUB 1 domain for capture, as described in Example 16. The results are shown in Figure 26.

The results of these experiments show that patients with PE (without or with DVT) have significantly elevated levels of ADAMTS13/FXI complex. The results demonstrate that quantitation of plasma ADAMTS13/FXI complex levels by ELISA is a useful indication of PE. The ELISA can also be useful for determining if a patient diagnosed with a DVT also has a PE. Other hemostatic disorders can be detected by following the methods described in this example. Both polyclonal and monoclonal antibodies to ADAMTS13 can be used for making the ELISA.

### Example 21. Characterization of ADAMTS13 Protein and FXI Protein comprising the ADAMTS13/FXI Complex in Plasma

ADAMTS13/FXI complex was removed from a citrated plasma sample containing high amounts of the complex by passing the plasma over an immunoaffinity column with goat anti-ADAMTS13 antibody conjugated to sepharose beads. After extensive washing with PBS, the bound complex was eluted from the column with 0.1 M glycine buffer pH 2.5. The eluent was dried by lyophilization, dissolved in SDS-sample buffer, and electrophoresed on a 4-20% SDS-PAGE polyacrylamide gel. RecADAMTS13 (see Figure 27, lane c) or Factor XI (see Figure 27, lane f) was applied to the gel as a positive control.

After electrophoresis, the proteins were transferred onto a nitrocellulose membrane and immunostained. Figure 27 shows lanes stained with a) anti-ADAMTS13 antibody; b) no primary antibody as control; c) recADAMTS13 positive control, stained with biotinlyated anti-ADAMTS13 antibody; d) anti-FXI IgG-HRP; e) no primary antibody; f) FXI positive control, stained with rabbit anti-fXI IgG-HRP. Lanes a-c were incubated with goat anti-rabbit IgG-HRP. The immunoreactive bands were detected by chemiluminescence.

As shown in Figure 27, the goat anti-ADAMTS13 antibody specifically immunoprecipitated a protein of approximately 80 kD reactive with the rabbit anti-ADAMTS13 antibody (arrow) that was not stained with the secondary antibody alone (lane b). RecADAMTS13 was immunostained (lane c). Similarly, a protein of approximately 160 kD in the immunoprecipitate from the goat ADAMTS13 antibody was specifically immunostained with anti-FXI antibody (lane d) but not with secondary antibody alone (lane e). FXI positive control was immunostained (lane f).

These results show that a fragment of ADAMTS13 of approximately 80 kD is co-immunoprecipitated with FXI by the goat anti-ADAMTS13 antibody. Not wishing to be bound by theory, the approximate amino acid sequence comprising the approximately 80kD fragment isolated from plasma in the ADAMTS13/FXI complex appears to encompass amino acids 566 to 1336 (SEQ ID NO: 5) of ADAMTS13 protein Q76LX8 (SEQ ID NO: 4) designated in the Swiss Protein data bank. This sequence includes the spacer domain through a small N-terminal portion of the CUB2 domain.

### Example 22. Generation of Antibodies Reactive with ADAMTS13/FXI Complex

Mice were immunized with recombinant full length human ADAMTS13 according to standard protocols. At an appropriate time after immunization, mice were sacrificed, the spleens removed and hybridomas made by fusion of spleen cells with appropriate mouse cells according to standard procedures. Cells were plated and grown in culture medium. Cell supernatants were harvested and tested for their ability to recognize ADAMTS13/FXI complex in an ELISA.

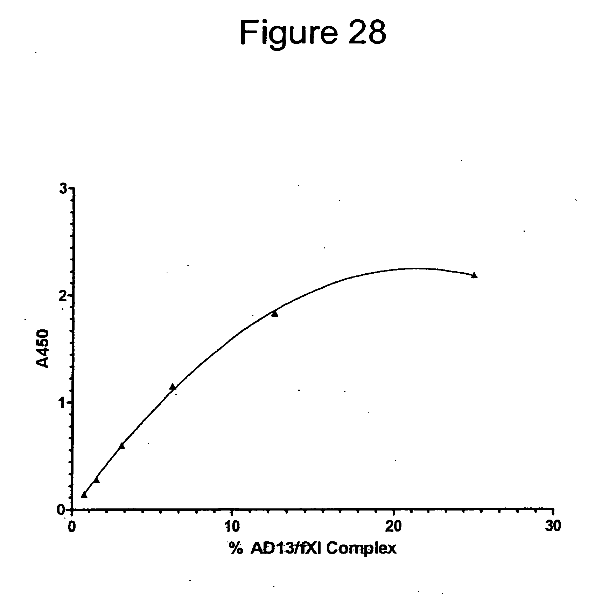

In particular, Mab 5 antibody was purified from culture supernatant and the purified monoclonal antibody was used to coat microtiter wells. Plasma containing ADAMTS13/FXI complex was added to the well at different concentrations. The ELISA procedure using anti-FXI-HRP antibody as detection antibody (described in Example 16) was performed. Wells having significant absorbance above background represent monoclonal antibodies that bind to ADAMTS13 in the ADAMTS13/FXI complex. The results are shown in Figure 28.

Mabs 4, 6, 7 and 12 were also selected using this methodology. Similarly, polyclonal anti-ADAMTS13 antibodies binding to ADAMTS13/FXI complex can be selected in the same manner. Polyclonal antibodies are generated by immunization of animals (*e.g*. mice, rabbits, sheep, goats) with full length or peptide fragments of recADAMTS13, native ADAMTS13 or by synthetic peptides homologous to specific sequences of ADAMTS13.

Alternatively, hybridoma supernatants can be screened using the solid phase immunoassay procedure described in Example 17. In this assay, FXI or FXIa is bound to the microtiter wells and ADAMTS13 protein is added to the well to form a FXI/ADAMTS13complex. Monoclonal antibodies or hybridoma cell culture fluid are added to the wells. The ELISA procedure is then completed as described in Example 17. Wells with significant color development represent monoclonal antibodies that bind to ADAMTS13 complexed with FXI.

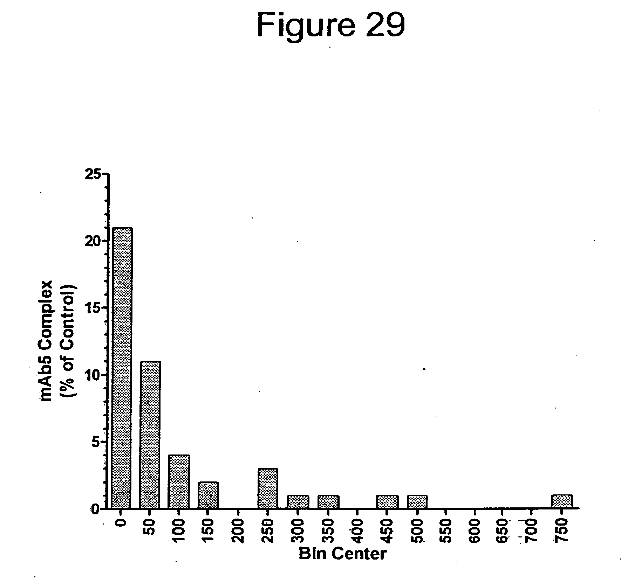

An ELISA using an anti-ADAMTS13 monoclonal antibody as capture can be used to measure ADAMTS13/FXI complexes in plasma. The quantitation of ADAMTS13/FXI complex in normal plasma samples using a monoclonal antibody as detection in the ELISA is shown in Figure 29.

Various modifications and variations of the described products and methods of the invention will be apparent to those skilled in the art without departing from the scope and spirit of the invention. Although the invention has been described in connection with specific preferred embodiments, it should be understood that the invention as claimed should not be unduly limited to such specific embodiments.

## Claims

1. A method for detecting a pulmonary embolism in an individual comprising:
a) adding a test plasma sample from the individual to a solid phase having an anti-ADAMTS13 antibody bound thereto, wherein ADAMTS13/FXI complexes present in the sample bind to the antibody;
b) adding an anti-FXI antibody to the solid phase, wherein the FXI antibody binds to FXI in ADAMTS13/FXI complexes;
c) detecting ADAMTS13/FXI complexes by direct or indirect immunolabelling of the anti-FXI antibody,
wherein an increase-in ADAMTS13/FXI complexes in the test plasma sample-compared to ADAMTS131FXI complexes in a control plasma sample having normal ADAMTS13 activity indicates a pulmonary embolism.

2. The method of claim 1, wherein the anti-ADAMTS13 antibody binds to an epitope in SEQ ID NO: 2.

3. The method of claim 1, wherein the solid phase is a membrane, plate, microwell or bead.

4. The method of claim 1, wherein detecting ADAMTS13/FXI complexes is by direct immunolabelling of the anti-FXI antibody.

5. The method of claim 4, wherein the anti-FXI antibody is labeled with horse radish peroxidase.

6. The method of claim 1, wherein detecting ADAMTS13/FXI complexes is by indirect immunolabelling of the anti-FXI antibody.

7. The method of claim 6, wherein the anti-FXI antibody is raised in a first species and wherein a labeled antibody raised in a second species against antibodies from the first species is added to the solid phase and detected.

## Patentansprüche

1. Verfahren zum Nachweisen von Lungenembolie in einem Individuum, umfassend:
a) Zugeben einer Plasmaprobe von dem Individuum zu einer Festphase mit einem daran gebundenen Anti-ADAMTS13-Antikörper, wobei ADAMTS13/FXI-Komplexe, die in der Probe vorhanden sind, an den Antikörper binden;
b) Zugeben eines Anti-FXI-Antikörpers zu der Festphase, wobei der FXI-Antikörper an FXI in ADAMTS13/FXI-Komplexen bindet;
c) Nachweisen von ADAMTS13/FXI-Komplexen durch direkte oder indirekte Immunomarkierung des Anti-FXI-Antikörpers,
wobei eine Zunahme an ADAMTS13/FXI-Komplexen in der Testplasmaprobe im Vergleich zu ADAMTS13/FXI-Komplexen in einer Kontrollplasmaprobe mit normaler ADAMTS13-Aktivität auf eine Lungenembolie hinweist.

2. Verfahren nach Anspruch 1, wobei der Anti-ADAMTS13-Antikörper an ein Epitop in SEQ ID NO: 2 bindet.

3. Verfahren nach Anspruch 1, wobei die Festphase eine Membran, Platte, Mikronapf oder Kügelchen ist.

4. Verfahren nach Anspruch 1, wobei das Nachweisen von ADAMTS13/FXI-Komplexen mittels direkter Immunomarkierung des Anti-FXI-Antikörpers erfolgt.

5. Verfahren nach Anspruch 4, wobei der Anti-FXI-Antikörper mit Meerrettichperoxidase markiert ist.

6. Verfahren nach Anspruch 1, wobei das Nachweisen von ADAMTS13/FXI-Komplexen mittels indirekter Immunomarkierung des Anti-FXI-Antikörpers erfolgt.

7. Verfahren nach Anspruch 6, wobei der Anti-FXI-Antikörper in einer Spezies gezogen wird, und wobei ein markierter Antikörper, der in einer zweiten Spezies gegen Antikörper aus der ersten Spezies gezogen worden ist, zu der Festphase zugegeben und nachgewiesen wird.

## Revendications

1. Procédé de détection d'embolie pulmonaire chez un individu, comprenant:
a) ajouter un échantillon de plasma à tester provenant de l'individu sur une phase solide à laquelle un anticorps anti-ADAMTS13 est lié, les complexes ADAMTS13/FXI présents dans l'échantillon se liant à l'anticorps ;
b) ajouter un anticorps anti-FXI à la phase solide, ledit anticorps anti-FXI se liant au FXI présent dans les complexes ADAMTS13/FXI ;
c) détecter les complexes ADAMTS13/FXI par immunomarquage direct ou indirect de l'anticorps anti-FXI,
dans lequel une augmentation des complexes ADAMTS13/FXI dans l'échantillon de plasma à tester par rapport aux complexes ADAMTS13/FXI présents dans un échantillon de plasma témoin ayant une activité d'ADAMTS13 normale indique une embolie pulmonaire.

2. Procédé selon la revendication 1, dans lequel l'anticorps anti-ADAMTS13 se lie à un épitope dans la SEQ ID N° 2.

3. Procédé selon la revendication 1, dans lequel la phase solide est une membrane, une plaque, un micropuits ou une bille.

4. Procédé selon la revendication 1, dans lequel la détection des complexes ADAMTS13/FXI se fait par immunomarquage direct de l'anticorps anti-FXI.

5. Procédé selon la revendication 4, dans lequel l'anticorps anti-FXI est marqué par la peroxydase de raifort.

6. Procédé selon la revendication 1, dans lequel la détection des complexes ADAMTS13/FXI se fait par immunomarquage indirect de l'anticorps anti-FXI.

7. Procédé selon la revendication 6, dans lequel l'anticorps anti-FXI est produit dans une première espèce et dans lequel un anticorps marqué produit dans une deuxième espèce contre des anticorps de la première espèce est ajouté à la phase solide et détecté.


### SEQUENCE LISTING

<110> GREENFIELD, ROBERT
   RANZURMAL, SAFI
   FRYER, HUGH
<120> METHODS AND KITS FOR DETECTING AND MEASURING
   ADAMTS13/FXI COMPLEXES
<130> 520001-2002.2
<140> 11/709,562
   <141> 2007-02-22
<150> 11/107,602
   <151> 2005-04-15
<150> 10/894,702
   <151> 2004-07-19
<160> 5
<170> PatentIn ver. 3.2
<210> 1
   <211> 12
   <212> PRT
   <213> Artificial Sequence
<220>
   <223> Description of Artificial Sequence: synthetic peptide
<220>
   <221> MOD_RES
   <222> (2)
   <223> Lys(DABCYL)
<220>
   <221> MOD_RES
   <222> (11)
   <223> Asp(EDANS)
<400> 1
   Arg Lys Asn Leu Val Tyr Met Val Thr Gly Asp Arg
   1 5 10
<210> 2
   <211> 4
   <212> PRT
   <213> Artificial Sequence
<220>
   <223> Description of Artificial sequence: Synthetic peptide
<400> 2
   Leu Leu Val Tyr
   1
<210> 3
   <211> 9
   <212> PRT
   <213> Artificial sequence
<220>
   <223> Description of Artificial sequence: Synthetic peptide
<400> 3
   Asn Leu Val Tyr Met Val Thr Gly Asp
   1 5
<210> 4
   <211> 1427
   <212> PRT
   <213> Homo sapiens
<400> 4
   Met His Gln Arg His Pro Arg Ala Arg Cys Pro Pro Leu Cys Val Ala
   1               5                   10                  15
   Gly Ile Leu Ala Cys Gly Phe Leu Leu Gly Cys Trp Gly Pro Ser His
               20                  25                  30
   Phe Gln Gln Ser Cys Leu Gln Ala Leu Glu Pro Gln Ala Val Ser Ser
           35                  40                  45
   Tyr Leu Ser Pro Gly Ala Pro Leu Lys Gly Arg Pro Pro Ser Pro Gly
       50                  55                  60
   Phe Gln Arg Gln Arg Gln Arg Gln Arg Arg Ala Ala Gly Gly Ile Leu
   65                  70                  75                  80
   His Leu Glu Leu Leu Val Ala Val Gly Pro Asp Val Phe Gln Ala His
                   85                  90                  95
   Gln Glu Asp Thr Glu Arg Tyr Val Leu Thr Asn Leu Asn Ile Gly Ala
               100                 105                 110
   Glu Leu Leu Arg Asp Pro Ser Leu Gly Ala Gln Phe Arg Val His Leu
           115                 120                 125
   Val Lys Met Val Ile Leu Thr Glu Pro Glu Gly Ala Pro Asn Ile Thr
       130                 135                 140
   Ala Asn Leu Thr Ser Ser Leu Leu Ser Val Cys Gly Trp Ser Gln Thr
   145                 150                 155                 160
   Ile Asn Pro Glu Asp Asp Thr Asp Pro Gly His Ala Asp Leu Val Leu
                   165                 170                 175
   Tyr Ile Thr Arg Phe Asp Leu Glu Leu Pro Asp Gly Asn Arg Gln Val
               180                 185                 190
   Arg Gly Val Thr Gln Leu Gly Gly Ala Cys Ser Pro Thr Trp Ser Cys
           195                 200                 205
   Leu Ile Thr Glu Asp Thr Gly Phe Asp Leu Gly Val Thr Ile Ala His
       210                 215                 220
   Glu Ile Gly His Ser Phe Gly Leu Glu His Asp Gly Ala Pro Gly Ser
   225                 230                 235                 240
   Gly Cys Gly Pro Ser Gly His Val Met Ala Ser Asp Gly Ala Ala Pro
   245 250 255
   Arg Ala Gly Leu Ala Trp Ser Pro Cys Ser Arg Arg Gln Leu Leu Ser
   260 265 270
   Leu Leu Ser Ala Gly Arg Ala Arg Cys Val Trp Asp Pro Pro Arg Pro
   275 280 285
   Gln Pro Gly Ser Ala Gly His Pro Pro Asp Ala Gln Pro Gly Leu Tyr
   290 295 300
   Tyr Ser Ala Asn Glu Gln Cys Arg Val Ala Phe Gly Pro Lys Ala Val
   305 310 315 320
   Ala Cys Thr Phe Ala Arg Glu His Leu Asp Met Cys Gln Ala Leu Ser
   325 330 335
   Cys His Thr Asp Pro Leu Asp Gln Ser Ser Cys Ser Arg Leu Leu Val
   340 345 350
   Pro Leu Leu Asp Gly Thr Glu Cys Gly Val Glu Lys Trp Cys Ser Lys
   355 360 365
   Gly Arg Cys Arg Ser Leu Val Glu Leu Thr Pro Ile Ala Ala Val His
   370 375 380
   Gly Arg Trp Ser Ser Trp Gly Pro Arg Ser Pro Cys Ser Arg Ser Cys
   385 390 395 400
   Gly Gly Gly Val Val Thr Arg Arg Arg Gln Cys Asn Asn Pro Arg Pro
   405 410 415
   Ala Phe Gly Gly Arg Ala Cys Val Gly Ala Asp Leu Gln Ala Glu Met
   420 425 430
   Cys Asn Thr Gln Ala Cys Glu Lys Thr Gln Leu Glu Phe Met Ser Gln
   435 440 445
   Gln Cys Ala Arg Thr Asp Gly Gln Pro Leu Arg Ser Ser Pro Gly Gly
   450 455 460
   Ala Ser Phe Tyr His Trp Gly Ala Ala Val Pro His Ser Gln Gly Asp
   465 470 475 480
   Ala Leu Cys Arg His Met Cys Arg Ala Ile Gly Glu Ser Phe Ile Met
   485 490 495
   Lys Arg Gly Asp Ser Phe Leu Asp Gly Thr Arg Cys Met Pro Ser Gly
   500 505 510
   Pro Arg Glu Asp Gly Thr Leu Ser Leu Cys Val Ser Gly Ser Cys Arg
   515 520 525
   Thr Phe Gly Cys Asp Gly Arg Met Asp Ser Gln Gln Val Trp Asp Arg
   530 535 540
   Cys Gln Val Cys Gly Gly Asp Asn Ser Thr Cys Ser Pro Arg Lys Gly
   545 550 555 560
   Ser Phe Thr Ala Gly Arg Ala Arg Glu Tyr Val Thr Phe Leu Thr Val
   565 570 575
   Thr Pro Asn Leu Thr Ser Val Tyr Ile Ala Asn His Arg Pro Leu Phe
   580 585 590
   Thr His Leu Ala Val Arg Ile Gly Gly Arg Tyr Val Val Ala Gly Lys
   595 600 605
   Met Ser Ile Ser Pro Asn Thr Thr Tyr Pro Ser Leu Leu Glu Asp Gly
   610 615 620
   Arg Val Glu Tyr Arg Val Ala Leu Thr Glu Asp Arg Leu Pro Arg Leu
   625 630 635 640
   Glu Glu Ile Arg Ile Trp Gly Pro Leu Gln Glu Asp Ala Asp Ile Gln
   645 650 655
   Val Tyr Arg Arg Tyr Gly Glu Glu Tyr Gly Asn Leu Thr Arg Pro Asp
   660 665 670
   Ile Thr Phe Thr Tyr Phe Gln Pro Lys Pro Arg Gln Ala Trp Val Trp
   675 680 685
   Ala Ala Val Arg Gly Pro Cys Ser Val Ser Cys Gly Ala Gly Leu Arg
   690 695 700
   Trp Val Asn Tyr Ser Cys Leu Asp Gln Ala Arg Lys Glu Leu Val Glu
   705 710 715 720
   Thr Val Gln Cys Gln Gly Ser Gln Gln Pro Pro Ala Trp Pro Glu Ala
   725 730 735
   Cys Val Leu Glu Pro Cys Pro Pro Tyr Trp Ala Val Gly Asp Phe Gly
   740 745 750
   Pro Cys Ser Ala Ser Cys Gly Gly Gly Leu Arg Glu Arg Pro Val Arg
   755 760 765
   Cys Val Glu Ala Gln Gly Ser Leu Leu Lys Thr Leu Pro Pro Ala Arg
   770 775 780
   Cys Arg Ala Gly Ala Gln Gln Pro Ala Val Ala Leu Glu Thr Cys Asn
   785 790 795 800
   Pro Gln Pro Cys Pro Ala Arg Trp Glu Val Ser Glu Pro Ser Ser Cys
   805 810 815
   Thr Ser Ala Gly Gly Ala Gly Leu Ala Leu Glu Asn Glu Thr Cys Val
   820 825 830
   Pro Gly Ala Asp Gly Leu Glu Ala Pro Val Thr Glu Gly Pro Gly Ser
   835 840 845
   Val Asp Glu Lys Leu Pro Ala Pro Glu Pro Cys Val Gly Met Ser Cys
   850 855 860
   Pro Pro Gly Trp Gly His Leu Asp Ala Thr Ser Ala Gly Glu Lys Ala
   865 870 875 880
   Pro Ser Pro Trp Gly Ser Ile Arg Thr Gly Ala Gln Ala Ala His Val
   885 890 895
   Trp Thr Pro Ala Ala Gly Ser Cys Ser Val Ser Cys Gly Arg Gly Leu
   900 905 910
   Met Glu Leu Arg Phe Leu Cys Met Asp Ser Ala Leu Arg Val Pro Val
   915 920 925
   Gln Glu Glu Leu Cys Gly Leu Ala Ser Lys Pro Gly Ser Arg Arg Glu
   930 935 940
   Val Cys Gln Ala Val Pro Cys Pro Ala Arg Trp Gln Tyr Lys Leu Ala
   945 950 955 960
   Ala Cys Ser Val Ser Cys Gly Arg Gly Val Val Arg Arg Ile Leu Tyr
   965 970 975
   Cys Ala Arg Ala His Gly Glu Asp Asp Gly Glu Glu Ile Leu Leu Asp
   980 985 990
   Thr Gln Cys Gln Gly Leu Pro Arg Pro Glu Pro Gln Glu Ala Cys Ser
   995 1000 1005
   Leu Glu Pro Cys Pro Pro Arg Trp Lys Val Met Ser Leu Gly Pro
   1010 1015 1020
   Cys Ser Ala Ser Cys Gly Leu Gly Thr Ala Arg Arg Ser Val Ala
   1025 1030 1035
   Cys Val Gln Leu Asp Gln Gly Gln Asp Val Glu Val Asp Glu Ala
   1040 1045 1050
   Ala Cys Ala Ala Leu Val Arg Pro Glu Ala Ser Val Pro Cys Leu
   1055 1060 1065
   Ile Ala Asp Cys Thr Tyr Arg Trp His Val Gly Thr Trp Met Glu
   1070 1075 1080
   Cys Ser Val Ser Cys Gly Asp Gly Ile Gln Arg Arg Arg Asp Thr
   1085 1090 1095
   Cys Leu Gly Pro Gln Ala Gln Ala Pro Val Pro Ala Asp Phe Cys
   1100 1105 1110
   Gln His Leu Pro Lys Pro Val Thr Val Arg Gly Cys Trp Ala Gly
   1115 1120 1125
   Pro Cys Val Gly Gln Gly Thr Pro Ser Leu Val Pro His Glu Glu
   1130 1135 1140
   Ala Ala Ala Pro Gly Arg Thr Thr Ala Thr Pro Ala Gly Ala Ser
   1145 1150 1155
   Leu Glu Trp Ser Gln Ala Arg Gly Leu Leu Phe Ser Pro Ala Pro
   1160 1165 1170
   Gln Pro Arg Arg Leu Leu Pro Gly Pro Gln Glu Asn Ser Val Gln
   1175 1180 1185
   Ser Ser Ala Cys Gly Arg Gln His Leu Glu Pro Thr Gly Thr Ile
   1190 1195 1200
   Asp Met Arg Gly Pro Gly Gln Ala Asp Cys Ala Val Ala Ile Gly
   1205 1210 1215
   Arg Pro Leu Gly Glu Val Val Thr Leu Arg Val Leu Glu Ser Ser
   1220 1225 1230
   Leu Asn Cys Ser Ala Gly Asp Met Leu Leu Leu Trp Gly Arg Leu
   1235 1240 1245
   Thr Trp Arg Lys Met Cys Arg Lys Leu Leu Asp Met Thr Phe Ser
   1250 1255 1260
   Ser Lys Thr Asn Thr Leu Val Val Arg Gln Arg Cys Gly Arg Pro
   1265 1270 1275
   Gly Gly Gly Val Leu Leu Arg Tyr Gly Ser Gln Leu Ala Pro Glu
   1280 1285 1290
   Thr Phe Tyr Arg Glu Cys Asp Met Gln Leu Phe Gly Pro Trp Gly
   1295 1300 1305
   Glu Ile Val Ser Pro Ser Leu Ser Pro Ala Thr Ser Asn Ala Gly
   1310 1315 1320
   Gly Cys Arg Leu Phe Ile Asn Val Ala Pro His Ala Arg Ile Ala
   1325 1330 1335
   Ile His Ala Leu Ala Thr Asn Met Gly Ala Gly Thr Glu Gly Ala
   1340 1345 1350
   Asn Ala Ser Tyr Ile Leu Ile Arg Asp Thr His Ser Leu Arg Thr
   1355 1360 1365
   Thr Ala Phe His Gly Gln Gln Val Leu Tyr Trp Glu Ser Glu Ser
   1370 1375 1380
   Ser Gln Ala Glu Met Glu Phe Ser Glu Gly Phe Leu Lys Ala Gln
   1385 1390 1395
   Ala Ser Leu Arg Gly Gln Tyr Trp Thr Leu Gln Ser Trp Val Pro
   1400 1405 1410
   Glu Met Gln Asp Pro Gln Ser Trp Lys Gly Lys Glu Gly Thr
   1415 1420 1425
<210> 5
   <211> 801
   <212> PRT
   <213> Homo sapiens
<400> 5
   Arg Ala Arg Glu Tyr Val Thr Phe Leu Thr Val Thr Pro Asn Leu Thr
   1               5                   10                  15
   Ser Val Tyr Ile Ala Asn His Arg Pro Leu Phe Thr His Leu Ala Val
               20                  25                  30
   Arg Ile Gly Gly Arg Tyr Val Val Ala Gly Lys Met Ser Ile Ser Pro
           35                  40                  45
   Asn Thr Thr Tyr Pro Ser Leu Leu Glu Asp Gly Arg Val Glu Tyr Arg
       50                  55                  60
   Val Ala Leu Thr Glu Asp Arg Leu Pro Arg Leu Glu Glu Ile Arg Ile
   65                  70                  75                  80
   Trp Gly Pro Leu Gln Glu Asp Ala Asp Ile Gln Val Tyr Arg Arg Tyr
                   85                  90                  95
   Gly Glu Glu Tyr Gly Asn Leu Thr Arg Pro Asp Ile Thr Phe Thr Tyr
               100                 105                 110
   Phe Gln Pro Lys Pro Arg Gln Ala Trp Val Trp Ala Ala Val Arg Gly
           115                 120                 125
   Pro Cys Ser Val Ser Cys Gly Ala Gly Leu Arg Trp Val Asn Tyr Ser
       130                 135                 140
   Cys Leu Asp Gln Ala Arg Lys Glu Leu Val Glu Thr Val Gln Cys Gln
   145                 150                 155                 160
   Gly Ser Gln Gln Pro Pro Ala Trp Pro Glu Ala Cys Val Leu Glu Pro
                   165                 170                 175
   Cys Pro Pro Tyr Trp Ala Val Gly Asp Phe Gly Pro Cys Ser Ala Ser
               180                 185                 190
   Cys Gly Gly Gly Leu Arg Glu Arg Pro Val Arg Cys Val Glu Ala Gln
           195                 200                 205
   Gly Ser Leu Leu Lys Thr Leu Pro Pro Ala Arg Cys Arg Ala Gly Ala
       210                 215                 220
   Gln Gln Pro Ala Val Ala Leu Glu Thr Cys Asn Pro Gln Pro Cys Pro
   225                 230                 235                 240
   Ala Arg Trp Glu Val Ser Glu Pro Ser Ser Cys Thr Ser Ala Gly Gly
                   245                 250                 255
   Ala Gly Leu Ala Leu Glu Asn Glu Thr Cys Val Pro Gly Ala Asp Gly
               260                 265                 270
   Leu Glu Ala Pro Val Thr Glu Gly Pro Gly Ser Val Asp Glu Lys Leu
           275                 280                 285
   Pro Ala Pro Glu Pro Cys Val Gly Met Ser Cys Pro Pro Gly Trp Gly
       290                 295                 300
   His Leu Asp Ala Thr Ser Ala Gly Glu Lys Ala Pro Ser Pro Trp Gly
   305                 310                 315                 320
   Ser Ile Arg Thr Gly Ala Gln Ala Ala His Val Trp Thr Pro Ala Ala
                   325                 330                 335
   Gly Ser Cys Ser Val Ser Cys Gly Arg Gly Leu Met Glu Leu Arg Phe
               340                 345                 350
   Leu Cys Met Asp Ser Ala Leu Arg Val Pro Val Gln Glu Glu Leu Cys
           355                 360                 365
   Gly Leu Ala Ser Lys Pro Gly Ser Arg Arg Glu Val Cys Gln Ala Val
       370                 375                 380
   Pro Cys Pro Ala Arg Trp Gln Tyr Lys Leu Ala Ala Cys Ser Val Ser
   385                 390                 395                 400
   Cys Gly Arg Gly Val Val Arg Arg Ile Leu Tyr Cys Ala Arg Ala His
                   405                 410                 415
   Gly Glu Asp Asp Gly Glu Glu Ile Leu Leu Asp Thr Gln Cys Gln Gly
               420                 425                 430
   Leu Pro Arg Pro Glu Pro Gln Glu Ala Cys Ser Leu Glu Pro Cys Pro
           435                 440                 445
   Pro Arg Trp Lys Val Met Ser Leu Gly Pro Cys Ser Ala Ser Cys Gly
   450 455 460
   Leu Gly Thr Ala Arg Arg Ser Val Ala Cys Val Gln Leu Asp Gln Gly
   465 470 475 480
   Gln Asp Val Glu Val Asp Glu Ala Ala Cys Ala Ala Leu Val Arg Pro
   485 490 495
   Glu Ala Ser Val Pro Cys Leu Ile Ala Asp Cys Thr Tyr Arg Trp His
   500 505 510
   Val Gly Thr Trp Met Glu Cys Ser Val Ser Cys Gly Asp Gly Ile Gln
   515 520 525
   Arg Arg Arg Asp Thr Cys Leu Gly Pro Gln Ala Gln Ala Pro Val Pro
   530 535 540
   Ala Asp Phe Cys Gln His Leu Pro Lys Pro Val Thr Val Arg Gly Cys
   545 550 555 560
   Trp Ala Gly Pro Cys Val Gly Gln Gly Thr Pro Ser Leu Val Pro His
   565 570 575
   Glu Glu Ala Ala Ala Pro Gly Arg Thr Thr Ala Thr Pro Ala Gly Ala
   580 585 590
   Ser Leu Glu Trp Ser Gln Ala Arg Gly Leu Leu Phe Ser Pro Ala Pro
   595 600 605
   Gln Pro Arg Arg Leu Leu Pro Gly Pro Gln Glu Asn Ser Val Gln Ser
   610 615 620
   Ser Ala Cys Gly Arg Gln His Leu Glu Pro Thr Gly Thr Ile Asp Met
   625 630 635 640
   Arg Gly Pro Gly Gln Ala Asp Cys Ala Val Ala Ile Gly Arg Pro Leu
   645 650 655
   Gly Glu Val Val Thr Leu Arg Val Leu Glu Ser Ser Leu Asn Cys Ser
   660 665 670
   Ala Gly Asp Met Leu Leu Leu Trp Gly Arg Leu Thr Trp Arg Lys Met
   675 680 685
   Cys Arg Lys Leu Leu Asp Met Thr Phe Ser Ser Lys Thr Asn Thr Leu
   690 695 700
   Val Val Arg Gln Arg Cys Gly Arg Pro Gly Gly Gly Val Leu Leu Arg
   705 710 715 720
   Tyr Gly Ser Gln Leu Ala Pro Glu Thr Phe Tyr Arg Glu Cys Asp Met
   725 730 735
   Gln Leu Phe Gly Pro Trp Gly Glu Ile Val Ser Pro Ser Leu Ser Pro
               740                 745                 750
   Ala Thr Ser Asn Ala Gly Gly Cys Arg Leu Phe Ile Asn Val Ala Pro
           755                 760                 765
   His Ala Arg Ile Ala Ile His Ala Leu Ala Thr Asn Met Gly Ala Gly
       770                 775                 780
   Thr Glu Gly Ala Asn Ala Ser Tyr Ile Leu Ile Arg Asp Thr His Ser
   785                 790                 795                 800
   Leu